(19) Europäisches Patentamt
European Patent Office
Office européen des brevets

(11) Publication number : 0 485 172 A2

(12) # EUROPEAN PATENT APPLICATION

(21) Application number : 91310225.7

(22) Date of filing : 05.11.91

(51) Int. Cl.[5] : **C08B 37/08,** C08B 11/00, C08F 8/00, C08G 69/48, A61K 31/735, A61K 31/765

(30) Priority : **06.11.90 US 609829**
**11.10.91 US 777429**

(43) Date of publication of application :
**13.05.92 Bulletin 92/20**

(84) Designated Contracting States :
**BE CH DE ES FR GB IT LI NL SE**

(71) Applicant : **FGN, INC.**
**Ventana Canyon Center, 5620 North Kolb Road**
**Tucson, Arizona 85715 (US)**
(71) Applicant : **THE UNIVERSITY OF ARIZONA**
**Tucson, Arizona 85721 (US)**

(72) Inventor : **Pamukcu, Rifat**
**1224 Herschel**
**Cincinatti, Ohio 45208 (US)**
Inventor : **Gross, Paul**
**126 West McKenzie**
**Stockton, California 95204 (US)**
Inventor : **Brendel, Klaus**
**3231 North Manor Drive**
**Tucson, Arizona 85715 (US)**

(74) Representative : **Baverstock, Michael George Douglas et al**
**BOULT, WADE & TENNANT 27 Furnival Street**
**London, EC4A 1PQ (GB)**

(54) **Esters and amides of substituted indenyl acetic acids.**

(57) Esters and amides of substituted indenyl acetic acids of the formula :

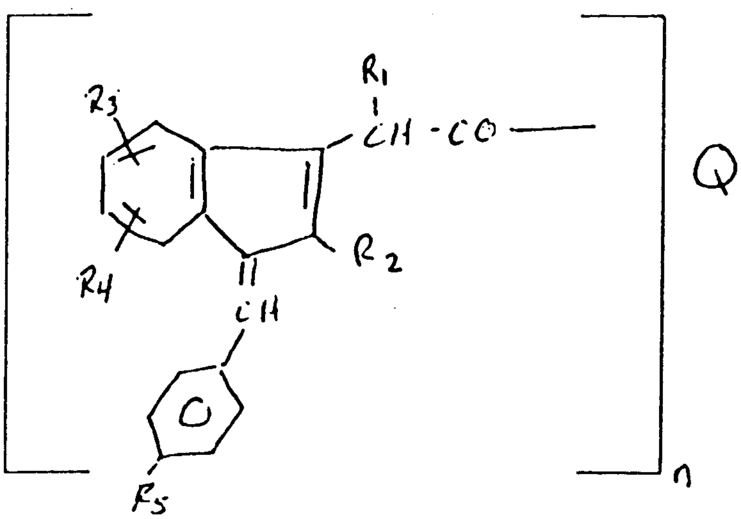

n is an integer of at least 2 ;

Q is a deprotonated residue of polymer or macromolecular structure having a molecular weight of at least about 1000 containing at least two primary and/or secondary amino groups and/or hydroxy groups ;

$R_1$ is selected from hydrogen, lower alkyl, or haloalkyl ;

$R_2$ is selected from hydrogen or alkyl ;

$R_3$ and $R_4$ are one or more members each independently chosen from hydrogen, alkyl, acyloxy, alkoxy, nitro, amino, acylamino, alkylamino, diakylamino, dialkylaminoalkyl, sulfamyl, alkythio, mercapto, hydroxy, hydroxyalkyl, alkylsulfonyl, halogen, cyano, carboxyl, carbalkoxy, carbamido, haloalkyl or cycloalkoxy ; and

$R_5$ is selected from alkylsulfenyl, alkylsulfinyl or alkylsulfonyl. are useful in the treatment of colonic polyps.

EP 0 485 172 A2

This invention relates to compounds and compositions for use in the treatment or prevention of colonic polyps.

Each year in the United States alone, approximately 60,000 people die from colon cancer, and over 150,000 new cases of colon cancer are diagnosed. For the American population as a whole, individuals have a six percent lifetime risk of developing colon cancer, making it the second most prevalent form of cancer in the country. Colon cancer is also prevalent in Western Europe.

To date, little progress has been made in the prevention and treatment of colorectal cancer, as reflected by the lack of change in the five-year survival rate over the last few decades. The only cure for this cancer is surgery at an extremely early stage. Unfortunately, most of these cancers are discovered too late for surgical cure, because most victims do not experience symptoms until the disease is advanced.

The incidence of colon cancer increases with age, particularly after the age of 40. Since the mean ages of populations in America and Western Europe are increasing, the prevalence of colorectal cancer should increase in the future.

In view of these grim statistics, efforts in recent years have concentrated on colon cancer prevention. Colon cancer usually arises from pre-existing benign growths known as polyps. Prevention efforts have emphasized the identification and removal of colonic polyps. Polyps are identified by x-ray and/or colonoscopy, and usually removed by devices associated with the colonoscope. The increased use of colon x-rays and colonoscopies in recent years has detected clinically significant precancerous polyps in four to six times the number of individuals per year that acquire colon cancer. During the past five years alone, an estimated 3.5 to 5.5 million people in the United States have been diagnosed with adenomatous colonic polyps, and it is estimated that many more people have or are susceptible to developing this condition, but are as yet undiagnosed. In fact, there are estimates that 10-12 percent of people over the age of 40 will form clinically significant adenomatous polyps.

Removal of polyps has been accomplished either with surgery or fiber-optic endoscopic polypectomy - - procedures that are uncomfortable, costly (the cost of a single polypectomy ranges between $1,000 and $1,500 for endoscopic treatment and more for surgery), and involve a small but significant risk of colon perforation. Overall, about £2.5 billion is spent annually in the United States in colon cancer treatment and prevention.

As indicated above, each polyp carries with it a chance that it will develop into a cancer. The likelihood of cancer is diminished if a polyp is removed. However, many of these patients demonstrate a propensity for developing additional polyps in the future. They must, therefore, be monitored periodically for the rest of their lives for polyp reoccurrence.

In most cases (i.e. the cases of so-called common sporadic polyps), polyp removal will be effective to reduce the risk of cancer. In a small percentage of cases (i.e. the cases of the so-called polyposis syndromes), removal of all or part of the colon is indicated. The difference between common sporadic polyps and polyposis syndromes is dramatic. Common sporadic polyp cases are characterized by relatively few polyps, each of which can usually be removed leaving the colon intact. By contrast, polyposis syndrome cases can be characterized by many (e.g. hundreds or more) of polyps- literally covering the colon in some cases -- making safe removal of the polyps impossible short of surgical removal of the colon. Because each polyp carries with it the palpable risk of cancerous development, polyposis syndrome patients invariably develop cancer if left untreated. Many of these patients have undergone a severe change in lifestyle as a result of the disfiguring surgery. Patients have strict dietary restrictions, and many must wear ostomy appliances to collect their intestinal wastes.

Recently, several non-steroidal anti-inflammatory drugs ("NSAIDs"), originally developed to treat arthritis, have shown effectiveness in inhibiting and eliminating polyps. Polyps virtually disappear when the patient take the drug. However, the prophylactic use of currently available NSAIDs, even in polyposis syndrome patients, is marked by severe side reactions that include gastrointestinal irritations and ulcerations. Once NSAID treatment is terminated due to such complications, the polyps return, particularly in polyposis syndrome patients.

This invention provides a novel class of compounds of formula I below that are effective in eliminating and inhibiting polyps, but are not characterized by the severe side reactions of NSAIDs. This invention also enables a method of treating patients with common sporadic polyps and polyposis syndromes to reduce or eliminate their polyps by administering to a patient in need of such treatment a physiologically effective amount of a compound of formula I.

As discussed above, the present invention is a class of compounds of formula I below,

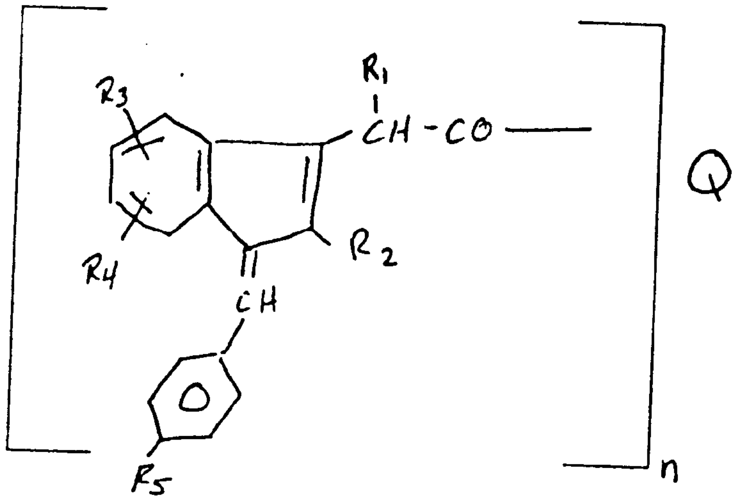

n is an integer of at least 2;

Q is a deprotonated residue of polymer or macromolecular structure having a molecular weight of at least about 1000 containing at least two primary and/or secondary amino groups and/or hydroxy groups;

$R_1$ is selected from hydrogen, lower alkyl, or haloalkyl;

$R_2$ is selected from hydrogen or alkyl;

$R_3$ and $R_4$ are one or more members each independently chosen from hydrogen, alkyl, acyloxy, alkoxy, nitro, amino, acylamino, alkylamino, diakylamino, dialkylaminoalkyl, sulfamyl, alkythio, mercapto, hydroxy, hydroxyalkyl, alkylsulfonyl, halogen, cyano, carboxyl, carbalkoxy, carbamido, haloalkyl, or cycloalkoxy; and

$R_5$ is selected from alkylsulfenyl, alkylsulfinyl or alkylsulfonyl.

As used herein, the term "halo" or "halogen" refers to chloro, bromo, fluoro and iodo groups, and the term "alkyl" refers to straight, branched or cyclic alkyl groups.

As used herein, the term macromolecule, macromolecular structure, or polymer refers to to molecules having at least two primary and/or secondary amino groups, and/or hydroxy groups. Examples of such amino-containing polymers or macromolecules are polyvinylamine, polyallylamine, polyethyleneimine, chitosan, polyamino acids, polyamine exchange resins (e.g. Amberlite) and polyaminoalkanes. Examples of hydroxy-containing polymers or macromolecules are polyhydroxyalkanes, polyvinylalcohols, carbohydrates (e.g. sucrose) and polyethylene glycols. The term "deprotonated residue" includes the situation where at least some, but not all, of the amino and/or hydroxy groups are deprotonated on the macromolecule or polymer.

The present invention also enables a method of treating individuals with common sporadic colonic polyps or polyposis syndrome by administering a pharmaceutically effective amount of a compound of formula I above where n, and $R_1$ - $R_5$ are as defined above, and Q is the deprotonated residue of a polyamine or polyhydroxy compound.

Compounds of this invention have unexpected utility for suppression of colonic polyps in view of the startling discovery that the effects of conventional NSAID therapy on colonic polyps can be, in fact, achieved via topical exposure to the agents. This effect was discovered in a patient with familial polyposis, a disease characterized by the presence of numerous colonic polyps. In an attempt to avoid colon cancer, the patient underwent surgical excision of the colon with formation of a continent ileostomy, or Kock's pouch. By this rarely performed surgical procedure, a pouch is constructed from the terminal portion of the small intestine. A colonic bacterial environment developed within the pouch resulting in extensive adenomatous polyp formation. Polyps also developed on the stoma, an external outlet from the pouch constructed from a contiguous portion of small intestine, and in the duodenum, the beginning of the small intestine. Sulindac, when administered in oral doses, led to the disappearance of the numerous polyps located in the pouch, but not the polyps on the stoma or in the duodenum. Given the understanding of the metabolic and excretory patterns of the drug as well as of bacterial enzyme activation of the agent, these rare findings suggested that high local concentrations of the drug were responsible for the effects in the pouch. It was apparent from the lack of response of the polyps in the other locations, particularly the stomal polyps which are close to the pouch, that the effect was topical and that blood-borne or systemic delivery of the drug was ineffectual.

The topical effect is particularly surprising since the cells believed to be responsible for polyp growth and subsequent malignancy are not only epithelial cells deep within the crypts of the intestine, but may also include cells which modulate the local immunological defense mechanisms in deeper mucosal and serosal layers of

the intestinal wall.

Compounds of this invention deliver active agents to the colon via the large macromolecular structure to which the active agent is conjugated. Colonic bacterial enzymes (or other colonic enzymes) cleave the active agent from the macromolecule, achieving locally high concentrations of the active agent and allowing the agent to contact the colon itself leading to inhibition of polyp proliferation.

The advantage of this treatment is that the active agent can be concentrated where it is effective, but whatever systemic levels are achieved are minimized. The systemic levels are particularly low because only passive absorption in the colon is involved. The negligible systemic levels are important in that the maintenance of chronic systemic levels of NSAIDs is complicated by a high incidence of gastric ulcers rendering them useless in a long-term prophylactic regimen.

Thus, contrary to prior approaches that relied on the high systemic levels of active agent to achieve the desired effect within the colon with the consequent gastric complications, the compounds of the present invention afford a different and safer therapeutic approach in light of the topical effect of these active agents on the colon itself.

Compounds of Formula I may be formulated into compositions together with pharmaceutically acceptable carriers for parenteral injection, for oral administration in solid or liquid form, for rectal administration, and the like, although oral administration is most preferred.

Compositions according to the present invention for parenteral injection may comprise pharmaceutically acceptable sterile aqueous or nonaqueous solutions, suspensions or emulsions. Examples of suitable nonaqueous carriers, diluents, solvents or vehicles include propylene glycol, vegetable oils, such as olive oil, and injectable organic esters such as ethyl oleate. Such compositions may also contain adjuvants such as preserving, wetting, emulsifying and dispersing agents. They may be sterilized, for example, by filtration through a bacteria-retaining filter, or by incorporating sterilizing agents into the compositions. They can also be manufactured in the form of sterile solid compositions which can be dissolved in sterile water, or some other sterile injectable medium immediately before use.

Solid dosage forms for oral administration include capsules, tablets, pills, powders, troches and granules. In such solid dosage forms, the active compound is admixed with at least one inert diluent such as sucrose, lactose or starch. Such dosage forms can also comprise, as is normal practice, additional substances other than diluents, e.g., lubricating agents such as magnesium stearate. In the case of capsules, tablets, troches and pills, the dosage forms may also comprise buffering agents. Tablets, pills and granules can additionally be prepared with enteric coatings.

Liquid dosage forms for oral administration include pharmaceutically acceptable emulsions, solutions, suspensions, syrups and elixirs containing inert diluents commonly used in the art, such as water. Besides such inert diluents, compositions can also include adjuvants such as wetting agents, emulsifying and suspending agents, and sweetening, flavoring and perfuming agents.

Compositions for rectal administration are preferably suppositories which may contain, in addition to the active substance, excipients such as cocoa butter or a suppository wax.

Actual dosage levels of active ingredient in the compositions of the invention may be varied so as to obtain an amount of active ingredient effective to achieve polyp-eliminating activity in accordance with the desired method of administration. The selected dosage level therefore depends upon the nature of the active compound administered, the route of administration, the desired duration of treatment, and other factors. If desired, the daily dose may be divided into multiple doses for administration, e.g. two to four times per day.

Compounds of this invention can be made by one of the five general schemes below.

SCHEME I

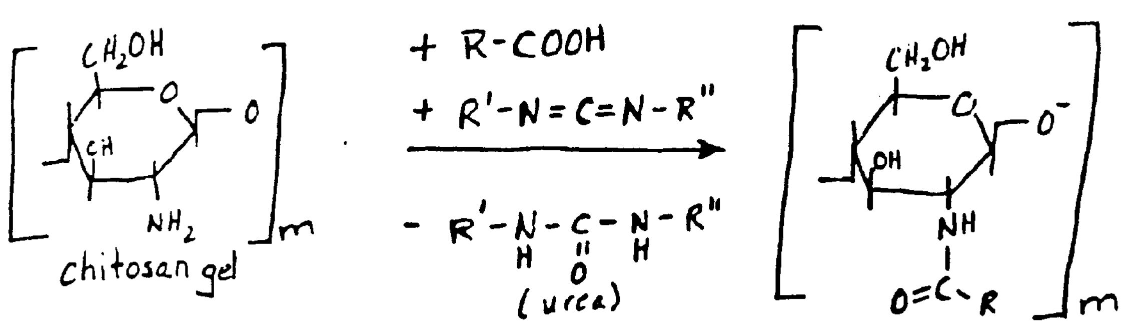

This scheme is useful for cases, where Q is water swellable polymer carrying aminogroups. The water soluble carbodiimide allows acylation in the alcoholic-aqueous phase, and the water soluble by-product urea can be removed by water, from the acylated polymer. The scheme allows acylation with carboxylic acid sensitive to the conditions of acid chloride or acid anhydride formation.

A chitosan gel $(GlcN)_m$ is prepared from chitosan, according to the method of S. Hirano et al. (Carbohyd. Res. 201 (1990) pp. 145-149) where m is the number of repeating units within the chitosan molecule. The gel is stirred in 70% aqueous methanol solution, at 0°-5° C, with the R-carboxylic acid (2 equivalents per GlcN) where R is the group in the brackets in formula I minus the attached carbonyl, and with a water-soluble carbodiimide (R′-N=C=N-R″; 2 equivalents per GlcN) for three days. (R′ and R″ are cycloalkyl or alkyl, and the like, containing also quaternary ammonium or sulfonate salt for solubilization of the carbodiimide.) The resulting gel is homogenized, washed well with distilled water, stirred with NaOH (1.2 equivalents per GlcN) in water (50 ml per g of chitosan) for five days. The mixture is homogenized and washed to neutrality. The gel is then dried to an amorphous powder.

SCHEME II

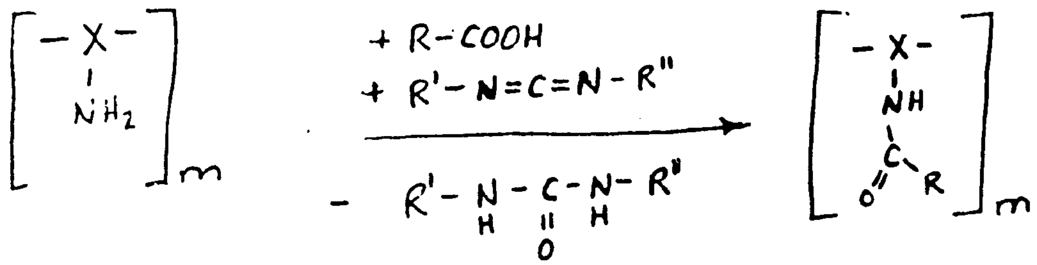

This scheme is useful for cases, where a salt between R-COOH and a polyamine Q is swellable or soluble in DMF. The carbodiimide is chosen, so that the by-product urea is soluble in dichloromethane, and therefore removable by extraction with it. Sodium hydroxide is used to extract unreacted R-COOH, so this scheme is useful for cases where acylation is difficult and incomplete. The scheme especially allows acylation with carboxylic acids that degrade under the conditions of acid chloride or acid anhydride formation.

$(GlcN)_m$ "chitosan," polylysine or similar polyamine "X" (0.01 mol-$NH_2$ groups; where m is the number of repeating amino-containing units per molecule of polyamine) are rapidly stirred in dimethylformanide ("DMF," 30 ml) at 50°C until no further dissolution is apparent. The cooled (0°) mixture is treated with carbodiimide (R′-N=C=N-R″; 0.011 mol) with continued stirring for two days. The resultant solution or suspension is poured into ice water. The precipitate is filtered off and washed with water. It is purified by being homogenized with and filtered from (a) $CH_2Cl_2$ (2x 50 ml); (b) 0.1 N-NaOH (2 x 50 ml); (c) 0.1N-HCl (2 x 50 ml); (d) $H_2O$ (2 x 50 ml); and (e) ether (2 x 50 ml). The resultant powder is dried.

SCHEME III

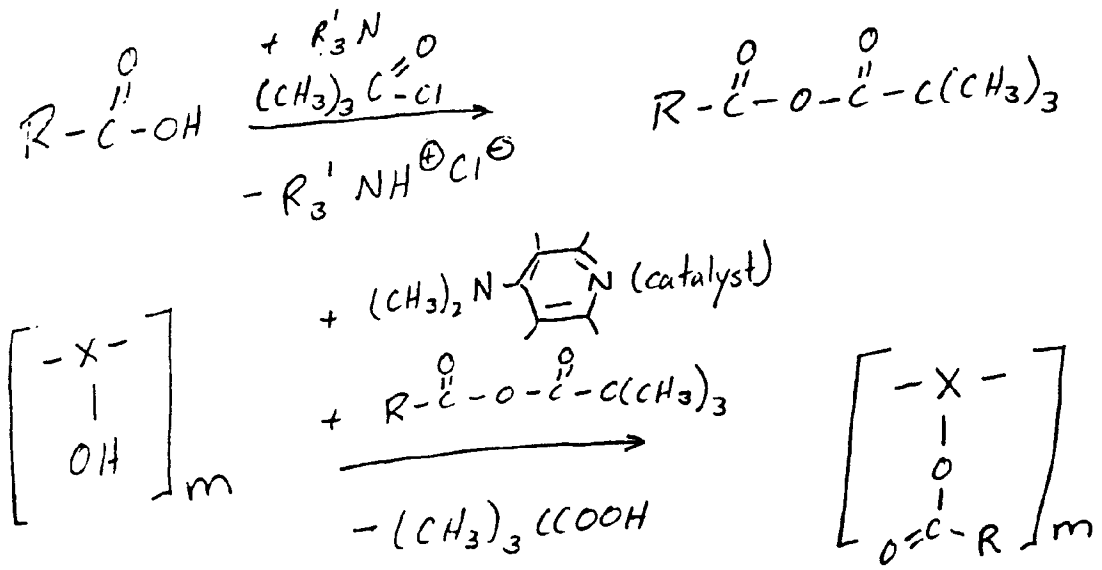


This scheme is suitable for cases were Q is a hydroxyl group-containing polymer that is soluble or swellable in dimethyl formamide. The bulky t-butyl group in pivalic acid prevents acylation by it, and dimethylamino pyridine catalyzes the difficult O-acylation. By-product pivalic acid is removable from the acylated polymer by extraction with organic solvent (e.g. toluene). The scheme is useful for carboxylic acids that are sensitive to the conditions of acylchloride synthesis.

Dry polyvinyl alcohol, methyl cellulose, or a similar swellable carbohydrate ($[X\text{-}OH]_m$; 0.01 mol-OH; where "m" is the number of repeating hydroxy-containing units in the polymeric compound) is rapidly stirred in absolute dimethyl formamide ("DMF," 50 ml) at 50°C until no further dissolution is apparent. Separately the carboxylic acid (RCOOH; 0.01 mol) is dissolved in absolute tetrahydrofuran (30 ml). At -10°C, pivaloyl chloride (0.01 mol) is added, followed by drop wise addition of a tertiary amine ($R'_3N$) (0.01 mol, e.g., triethylamine, ethyl diisopropylamine). The precipitated amine hydrochloride is filtered off. The solution is added, drop by drop, to the stirred and cooled (-10°C) polyol or carbohydrate mixture. The combined mixture is treated at -10°C with p-dimethyl amino pyridine (0.0001 mol.), and is allowed to come to room temperature and stay there for 15 hours. Toluene (100 ml) is added, with stirring. The mixture is evaporated to dryness in a rotary evaporator. The residue is homogenized in and filtered from (a) toluene (100 ml) and (b) water (2 x 100 ml). The filtercake is dried in vacuo at 40°C to constant weight.

SCHEME IV

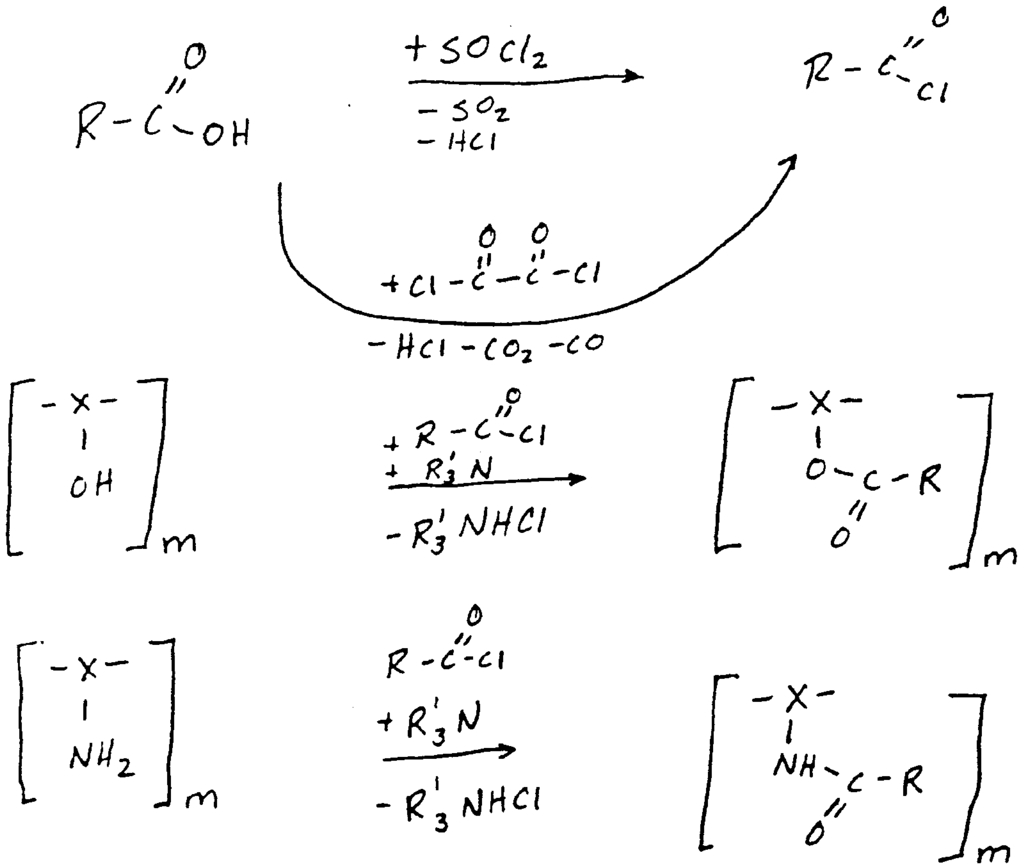


This scheme is useful for cases where Q is a polymer swellable in dimethyl formamide (DMF), and where it needs a highly reactive reagent for acylation. The scheme is suitable for carboxylic acids that form stable acid chlorides.

Carboxylic acid (R-COOH; 0.01 mol) is refluxed with thionylchloride or oxalylchloride (20 ml) until solution is complete and gas evolution ceases. Excess reagent is removed by evaporation. The residual acid chloride is diluted with tetrahydrofuran (10 ml) to give solution A.

A polyamine ($[-X-NH_2]_m$) such as chitosan, aminoethyl cellulose, polylysine (0.01 mol-$NH_2$), or a polyhydroxy compound ($[-X-OH]_m$) such as polyvinyl alcohol or a carbohydrate (e.g. methyl cellulose; 0.01 mol-OH) are heated in absolute dimethyl formamide at 50°C, until no further dissolution is apparent. Pyridine (0.001 mol) and p-dimethylaminopyridine (0.01 mol) are added. The mixture is cooled to -10°C, and solution A is added slowly with stirring. After 15 hours at room temperature, toluene (100 mol) is added, and the solution is evaporated in vacuo. The residue is homogenized with and filtered from (a) water (2 x 100 ml); (b) ether (2 x 100 ml), and is dried.

SCHEME V

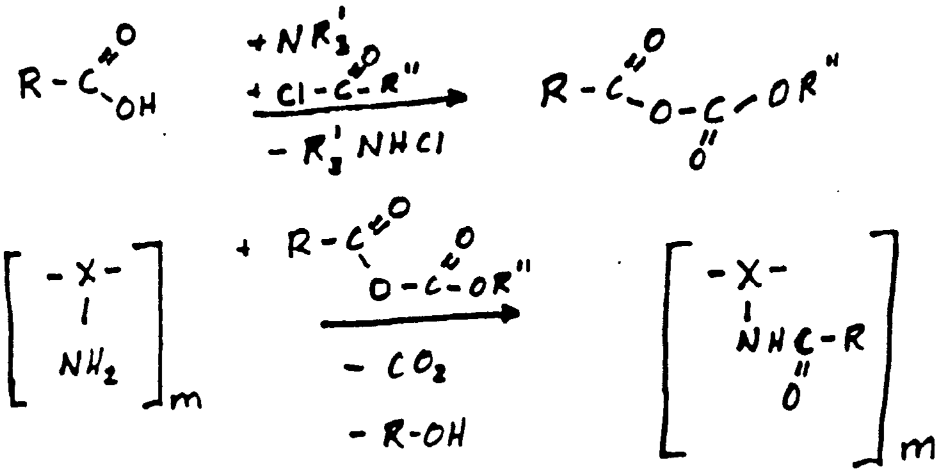


This scheme is useful, where Q is a polyamine swellable or soluble in dimethylformamide. It is especially suitable for cases where the removal of by-products such as salts, acids, or bases from the final product is difficult, since in this case only carbon dioxide and low molecular weight alcohol are produced as by-products. The scheme is especially useful for carboxylic acids that decompose under the conditions of acid chloride synthesis.

Chitosan, amino ethyl cellulose, polylysine or a similar polyamine ($[-X-NH_2]_m$; 0.01 mol $-NH_2$ groups) is rapidly stirred in dimethyl formamide (30 ml) at 50°C until no further dissolution is apparent. The carboxylic acid (RCOOH; 0.01 mol) is dissolved in absolute tetrahydrofuran (30 ml). At -20°C, first trialkylamine ($NR'_3$; 0.01 mol), and then alkylchlorocarbonate (Cl-COOR"; 0.01 mol, where R" is ethyl or isobutyl) is added. The precipitated trialkylammonium chloride ($R'_3NHCl$) is filtered off. The filtrate is added, with stirring, to the cold (-30°C) polyamine solution. After being stored for 15 hours at -15°C, the mixture is poured on ice (300 g), with stirring. After the ice has melted, the precipitate is filtered off, is thoroughly washed with water, and is dried.

The foregoing may be better understood from the following examples, which are presented for purposes of illustration and are not intended to limit the scope of the invention. As used in the following examples, the references to compounds such as (1), (2), (3) etc., and to substituents such as R, $R_1$, $R_2$ etc., refer to the corresponding compounds and substituents in the foregoing reaction schemes and in formula I.

## EXAMPLE 1

### Poly [5-Methoxy-2-Methyl-1-(ρ-Methylsulfinylbenzylidene)-3-Indenyl Acetyl] Chitosan

(A) α-Methyl-β-(ρ-methylthiophenyl) propionic acid.

To a solution of 2.3 g. (0.1 mole) of sodium in 100 ml. of absolute alcohol is added 17.4 g. (0.1 mole) of diethyl methylmalonate and 17.3 g. (0.1 mole) of ρ-methylthiobenzylchloride. The mixture is heated under a reflux in a water bath for three hours. The reaction mixture is poured into water, and the aqueous solution is extracted six times with ether and dried. It is then evaporated to yield diethyl methyl-ρ-methylthiobenzyl malonate. The crude product is then saponified by heating with excess 4% sodium hydroxide in aqueous ethanolic solution. The solution thus formed is concentrated, extracted with ether to remove any neutral material, and acidified with dilute sulfuric acid. The acidic mixture is heated on a steam bath for one hour, cooled and then extracted with ether. Evaporation of the ether solution gives α-methyl-β-(ρ-methylthiophenyl) propionic acid.

In a similar manner, using other substituted malonic esters in place of diethyl methylmalonate and other substituted benzyl halides in place of ρ-methylthiobenzyl chloride, the corresponding substituted propionic acids are obtained, for example:

α-methyl-β-(ρ-methoxyphenyl)propionic acid,
α-allyl-β-(ρ-nitrophenyl)propionic acid.

(B) 6-methoxy-2-methylindanone

α-Methyl-β-(ρ-methoxyphenyl)propionic acid (15 g.) is added to polyphosphoric acid (170 g.) at 50°C. and the mixture is heated at 83-90° for two hours. The syrup is poured into iced water, stirred for one-half hour, and then extracted with ether three times. The ether solution is washed with water twice, and with 5% $NaHCO_3$ five times until all the acidic material has been removed. The remaining neutral solution is washed with water and dried over sodium sulfate. Evaporation of the solution gives the indanone as a pale yellow oil.

In a similar manner, other β-aryl propionic acid compounds are converted to the corresponding indanone by the procedure of this example.

(C) Methyl 5-methoxy-2-methyl-3-indenylacetate.

A solution of 13.4 g. of 6-methoxy-2-methylindanone and 19.3 g. of methyl bromoacetate in 45 ml benzene is added over a period of 5 minutes to 21 g. of zinc amalgam (prepared according to Org. Syn. Coll., vol. 3) in 110 ml. benzene and 40 ml. dry ether. A few crystals of iodine are added to start the reaction, and the reaction mixture is maintained at reflux temperature (ca. 65°) with external heating. At 3 hour intervals, two batches of 10 g. zinc amalgam and 10g. bromoester are added, and the mixture is then refluxed for 8 hours. After addition of 30 ml. ethanol and 150 ml. of acetic acid, the mixture is poured into 700 ml. of 1:1 aqueous acetic acid. The organic layer is separated, and the aqueous layer is extracted twice with ether. The combined organic layers are washed thoroughly with water, ammonium hydroxide and water. Drying over sodium sulfate, evaporation of solvent in vacuo followed by pumping at 80° (bath temp.) (1-2 mm.) gives crude methyl(1-hydroxy-2-methyl-6-methoxy-indenyl)acetate.

A mixture of the above crude hydroxyester, 20 g. of ρ-toluenesulfonic acid monohydrate and 20 g. of anhydrous calcium chloride in 250 ml. toluene is refluxed overnight. The solution is filtered, and the solid residue is washed with benzene. The combined benzene solution is washed with water, sodium bicarbonate, water and then dried over sodium sulfate. After evaporation, the crude methyl 5-methoxy-2-methyl-3-indenylacetate is chromatographed on acid-washed alumina, and the product is eluted with petroleum ether-ether (v./v. 50-100%).

METHYL 2,6-DIMETHYL-3-INDENYLACETATE

The above reactions of Example 1C are repeated except that the starting materials are 2,5-dimethylindanone and methylbromoacetate. Using the same reaction conditions and techniques there is obtained methyl 2,6-dimethyl-3-indenylacetate.

The above reactions of Example 1C are repeated except that the starting materials are 6-methylthiolindanone and methylbromoacetate. Using the same reaction conditions and techniques, there is obtained methyl 5-methyl-thio-2-methyl-3-indenylacetate.

When any of the other indanones described in the other examples of the specification are used in the above procedure in place of 6-methoxy-2-methylindanone the corresponding methyl ester is obtained.

(D) 5-methoxy-2-methyl-1-(ρ-methylthiobenzylidene)-3-indenyl acetic acid.

To a solution of methyl 5-methoxy-2-methyl-3-indenylacetate 8.7 g. (0.037 mole) and ρ-methylthiobenzaldhyde, 6.3 g. (1.1 equivalent) is added 16+ ml. (2.0+ equivalents) of 25% methanolic sodium methoxide. The mixture is stirred at reflux under nitrogen for 2 hours. An equal volume of water is added dropwise and refluxing continues for 30 min. The solution is cooled, diluted with water and extracted with ether (3X). Residual ether is blown off with nitrogen, and then the aqueous solution is acidified with 50% glacial acetic acid. The precipitated product is collected and washed thoroughly with water. The crude product is crystallized from methanol to give pure 5-methoxy-2-methyl-1-(ρ-methylthiobenzylidene)-3-indenyl acetic acid (M.P. 195-196°).

5-METHOXY-2-METHYL-1-(ρ-ETHYLTHIOBENZYLIDENE) -3-INDENYL ACETIC ACID.

The above reaction of Example 1D is repeated using ρ-ethylthiobenzaldehyde instead of ρ-methylthiobenzaldehyde. Using the same reaction conditions and techniques, there is obtained 5-methoxy-2-methyl-1-(ρ-ethylthiobenzylidene)-3-indenyl acetic acid.

5-HYDROXY-2-METHYL-1-(ρ-METHYLTHIOBENZYLIDENE)-3-INDENYL ACETIC ACID

The reaction of Example 1D is repeated except that the starting materials are methyl 5-hydroxy-2-methyl-3-indenylacetate and ρ-methylthiobenzaldehyde. Using the same reaction conditions and techniques, there is obtained 5-hydroxy-2-methyl-1-(ρ-methylthiobenzylidene)-3-indenyl acetic acid.

The other methyl esters of Example 1C are reacted with ρ-methylthiobenzaldehyde according to the above procedure to produce the corresponding indenyl acetic acid.

(E) 5-methoxy-2-methyl-1-(ρ-methylsulfinylbenzylidene)-3-indenyl acetic acid.

A solution of sodium periodate (0.214 g.; 0.001 mole) in 3 ml. of water is added dropwise to 5-methoxy-2-methyl-1-(ρ-methylthiobenzylidene)-3-indenyl acetic acid (0.352 g.) (0.001 mole) in 25 ml. methanol and enough acetone to cause solution. This solution is stirred overnight at room temperature and filtered. The filtrate is evaporated at 30° to a sufficiently small volume that causes the product to precipitate. The suspension is diluted with several volumes of water, cooled and collected. The product is dried in vacuo over potassium hydroxide pellets and then in a vacuum oven at 70° to give 5-methoxy-2-methyl-1-(ρ-methylsulfinylbenzylidene)-3-indenyl acetic acid (M.P. 200.5-203.5°).

5-methoxy-2-methyl-1-(ρ-methylsulfonylbenzylidene)-3-indenyl acetic acid is prepared by the addition of 1.0 mole of m-chloroperbenzoic acid per mole of 5-methoxymethyl-1-(ρ-methylsulfinylbenzylidene)-3-indenyl acetic acid in an acetone solution.

(F) Poly [5-Methoxy-2-Methyl-1-(ρ-Methylsulfinylbenzylidene)-3-Indenyl Acetyl] Chitosan

5-methoxy-2-methyl-1-(ρ-methylsulfinylbenzylidene)-3-indenyl acetic acid (0.02 mol.) is conjugated to chitosan gel (0.01 mol. - $NH_2$) according to Scheme I. The procedure yields the desired compound ($R_1$ = hydrogen; $R_2$ = $CH_3$; $R_3$ = 5-$OCH_3$; $R_4$ = hydrogen; $R_5$ = p-$CH_3SO$; m > 50; n/m > 0.2; n > 10.)

Similarly, poly-5-methoxy-2-methyl-1-(ρ ethylsulfinylbenzylidene)-3-indenyl acetyl chitosan and poly-5-hydroxy-2-methyl-1-(ρ-methylsulfinylbenzylidene)-3-indenyl acetyl chitosan can be produced following the reaction of Example 1F using the appropriate thio-indenylacetic acids from Example 1D that have previously been converted to the corresponding sulfinylindenyl acetic acids according to the reaction of Example 1E.

EXAMPLE 2

Poly [5-Methoxy-2-Methyl-1-(ρ-Methylsulfinylbenzylidene)-3-Indenyl Acetyl] Polylysine

(A) 6-methoxy-2-methylindanone.

In a 500 ml. 3-necked flask is placed 36.2 g. (0.55 mole) of zinc dust, and in a 250 ml. addition funnel is charged a solution of 80 ml. anhydrous benzene, 20 ml. of anhydrous ether, 80 g. (0.58 mole) of ρ-anisaldehyde and 98 g. (0.55 mole) of ethyl-2-bromopropionate. About 10 ml. of the solution is added to the zinc dust with vigorous stirring, and the mixture is warmed gently until an exothermic reaction commences. The remaining reactants are added dropwise at such a rate that the reaction mixture is refluxing smoothly on its own accord (ca. 30-35 min.) After addition is completed, the mixture is placed in a water bath and refluxed for 30 minutes. After cooling to 0°, 250 ml. of 10% sulfuric acid is added with vigorous stirring. The benzene layer is extracted twice with 50 ml. portions of 5% sulfuric acid, and washed twice with 50 ml. portions of 5% sulfuric acid and washed twice with 50 ml. portions of water. The aqueous acidic layers are combined and extracted with 2 X 50 ml. ether. The combined etherel and benzene extracts are dried over sodium sulfate. Evaporation of solvent and fractionation of the residue through a 6″ Vigreux column affords the product, ethyl-2-hydroxy-(ρ-methoxyphenyl)-1-methylpropionate, B.P. 155-160° (1.5 mm).

By the method described in Vanden Zanden, Rec. trav. chim., 68, 413 (1949), the above compound is converted to 6-methoxy-2-methylindanone.

5-ETHYL-2-METHYLINDANONE

The above reactions of Example 2A are repeated except that the starting materials are o-ethylbenzaldehyde and ethyl-2-bromopropionate. Using the same reaction conditions and techniques, there is obtained 5-ethyl-2-methylindanone.

When the benzaldehydes listed in Table I below are utilized in the procedure of Example 2A, the corre-

sponding indanone is obtained.

Table I

| Aldehyde | Indanone |
|---|---|
| ρ-o-, or m-tolualdehyde | 2, 6-dimethyl, 2, 5-dimethyl, or 2, 4-dimethylindanone |
| ρ-o-, or m-hydroxybenzaldehyde | 4, 5 or 6-hydroxy-2-methylindanone |
| ρ-o-, or nitrobenzaldhyde | 2-methyl-(4, 5 or 6) nitroindanone |
| ρ-o-, or m-chlorobenzaldehyde | (4, 5, or 6) chloro-2-methylindanone |
| ρ-o-, or m-cyanobenzaldehyde | (4, 5, or 6) cyano-2 methylindanone |
| Vanillin | 6-hydroxy-5-methoxy 2-methylindanone |
| ρ-o-, or m-sulfamylbenzaldehyde | 2-methyl-(4,5 or 6-sulfamylindanone |
| 3-chloro-4-methylbenzaldehyde | 5-chloro-2,6 dimethylindanone |
| 4-carbamide-5-methylbenaldehyde | 6-carbomide-2,5 dimethylindanone |
| 3,4-difluorobenzaldhyde | 5, 6 difluoro-2-methylindanone |

(B) 5-methoxy-2-methyl-1-(ρ-methylsulfinylbenzylidene) -3-indenyl acetic acid

The reactions of Examples 1C, 1D and 1E are repeated, and 5-methoxy-2-methyl-1-(ρ-methylsulfinylbenzylidene)-3-indenyl acetic acid is obtained.

(C) Poly-5-methoxy-2-methyl-1-(ρ-methylsulfinylbenzylidene)-3-indenyl acetyl polylysine)

5-methoxy-2-methyl-1-(ρ-methylsulfinylbenzylidene)-3-indenyl acetic acid (0.01 mol.) is conjugated to polylysine (0.01 mol-$NH_2$) according to Scheme II. The procedure yields the desired compound ($R_1$ = hydrogen; $R_2$ = $CH_3$; $R_3$ = 5-O-$CH_3$; $R_4$ = hydrogen; $R_5$ = ρ-$CH_3SO$; Q = polylysine; m > 50; n/m > 0.2; n > 10.)

Similarly, if the reactions of Examples 1C-1E are repeated using 5-ethyl-2-methylindanone or any of the indanones in Table I, the corresponding polyindenyl acetyl polylysines are obtained.

EXAMPLE 3

Poly-α-[1-(ρ-Methylsulfonylbenzylidene-2-Methyl-5-Methoxy-3-Indenyl]-Propionyl Chitosan

(A) Methyl-α (5-methoxy-2-methyl-3-indenyl) propionate.

The procedure of Example 1C is followed using methyl α-bromopropionate in equivalent quantities in place of methyl bromoacetate used therein. There is obtained methyl α-(1-hydroxy-6-methoxy-2-methyl-1-indenyl)propionate, and it is then dehydrated to methyl α-(5-methoxy-2-methyl-3-indenyl)propionate in the same manner.

(B) α-[1-(ρ-methylthiobenzylidene)-2-methyl-5-methoxy-3-indenyl propionic acid

To a solution of 0.5 g. (0.00192 mole) of methyl α-(5-methoxy-2-methyl-3-indenyl) propionate and 0.595 g. (0.0039 mole) of ρ-methylthiobenzaldehyde in 3 ml. of anhydrous pyridine is added 1.63 g. of a 40% solution of benzyltrimethylammonium hydroxide (Triton-B) in methanol. The resulting red-purple solution is stirred at room temperature overnight.

The reaction mixture is poured into a mixture of ice and water, acidified with 2.5 N HCl, and extracted with ether. The ether solution is then washed with 2.5 N HCl until the washing acidifies (once), then with water until neutral. The ether layer is then extracted with 5% $Na_2CO_3$ solution. The $Na_2CO_3$ solution is washed with ether, acidified and extracted with ether. The ether solution is washed with water, dried over $Na_2SO_4$ and concentrated in vacuo to a yellow oil that foams up to a clear yellow solid on pumping at 0.5-1 mm. Thin layer chromatography of the product shows only one spot when eluted with a (v./v. 4:3:5) of isopropanol: 10% $NH_4OH$: ethyl acetate mixture;

U.V. absorption: >max, 3525, 2910, 2540, 2450. E%, 399, 260, 510 and 498.

(C) α-[1-(ρ-methylsulfinylbenzylidene)-2-methyl 5-methoxy-3-indenyl]-propionic acid

The procedure of Example 1E is followed using α-[1-(ρ-methylthiobenzylidene)-2-methyl-5-methoxy-3-indenyl]-propionic acid in place of 5-methoxy-2-methyl-1-(ρ-methylthiobenzylidene)-3-indenyl acetic acid thereby producing α-[1-(ρ-methylsulfinylbenzylidene)-2-methyl-5-methoxy-3-indenyl]-propionic acid (M.P. 115-120°).

α-[1-(ρ-methylsulfonylbenzylidene-2-methyl-5-methoxy-3-indenyl]-propionic acid is produced by the addition of 1.0 mole of m-chloroperbenzoic acid per mole of α[1-(ρ-methylsulfinylbenzylidene)-2-methyl-5-methoxy-3-indenyl]-propionic acid as described in Example 1E.

(D) Poly-α-[1-(ρ-methylsulfonylbenzylidene-2-methyl-5-methoxy-3-indenyl]-propionyl chitosan

α-[1-(ρ-methylsulfonylbenzylidene)-2-methyl-5-methoxy-3-indenyl]-propionic acid (0.02 mol.) is conjugated to chitosan gel (0.01 mol-$NH_2$) according to Scheme I. The procedure yields the desired compound ($R_1$ = $CH_3$; $R_2$ = $CH_3$; $R_3$ = 5-methoxy; $R_4$ = hydrogen; $R_5$ = $CH_3SO_2$-; Q = chitosan; m > 50; n/m > 0.2; n > 10.)

EXAMPLE 4

Poly-1-ρ-Methylsulfonylbenzylidene-5-Dimethylamino-3-Indenyl Acetyl Polylysine

(A) Methyl-3-hydroxy-2-methyl-5-nitro-3-indenylacetate

The procedure of Example 1C is followed using 2-methyl-6-nitro indanone in equivalent quantities in place of 6-methoxy-2-methyl-indanone used therein. After the mixture is condensed, 30 ml. of ethanol and 50 ml. of acetic acid are added. The mixture is then poured into 700 ml. of water. Extraction with ether gives methyl 3-hydroxy-2-methyl-5-nitro-3-indenylacetate.

(B) Methyl 5-dimethylamino-2-methyl-3-indenylacetate.

A solution of 0.05 mole of methyl 3-hydroxy-2-methyl-5-nitro-3-indenylacetate, 0.2 mole of 38% aqueous formaldehyde and 2 ml. of acetic acid in 100 ml. ethanol is reduced catalytically in the presence of a 10% Pd/C catalyst under 40 lb. p.s.i. hydrogen pressure at room temperature. The solution is filtered, evaporated and chromatographed on 300 g. of silica gel to give methyl 5-dimethylamino-3-hydroxy-2-methyl-3-indenylacetate.

The hydroxy ester is then dehydrated to methyl 5-dimethylamino-2-methyl-3-indenylacetate.

(C) 1-ρ-methylthiobenzylidene-5-dimethylamino -2-methyl-3-indenyl acetic acid.

To a solution of 2.5 g. of the ester from Part B of this example in 15 ml. of 1,2-dimethoxyethane at 0° is added 1.5 g. of ρ-methylthiobenzaldehyde followed by 1.1 g. of potassium t-butoxide. The reaction mixture is kept in the ice-bath for 4 hours, and then allowed to stand at room temperature for 18 hours. The mixture is diluted with 15 ml. of ether and the potassium salt is filtered. The salt is dissolved in 30 ml. of water and neutralized with dilute hydrochloric acid to pH 6-6.5. The crude acid precipitated is collected by filtration and chromatographed on a silica gel column, using ether-petroleum ether (v./v. 50-100%) as eluent to give pure 1-ρ-methylthiobenzylidene-5-dimethylamino-2-methyl-3-indenyl acetic acid which may be oxidized to 1-ρ-methylsulfinylbenzylidene-5-dimethylamino-2-methyl-3-indenyl acetic acid and 1-ρ-methylsulfonylbenzylidene-5-dimethylamino-2-methyl-3-indenyl acetic acid as described above.

(D) Poly [1-ρ-methylsulfonylbenzylidene-5-dimethylamino-3-indenyl acetyl] polylysine

1-ρ-methylsulfonylbenzylidene-5-dimethylamino-3-indenyl acetic acid (0.01 mol.) is conjugated to polylysine (0.01 mol-$NH_2$) according to Scheme II. The procedure yields the desired compound ($R_1$ = hydrogen; $R_2$ = $CH_3$; $R_3$ = 5-$N(CH_3)_2$; $R_4$ = hydrogen; $R_5$ = $CH_3SO_2$-; m > 50; n/m > 0.2; n > 10.)

EXAMPLE 5

Poly [α-(1-ρ-Methylsulfinylbenzylidene)-2-Methyl-5-Dimethylamino-3-Indenyl-Propionyl] Chitosan

(A) α-[1-(ρ-methylsulfinylbenzylidene)-2-methyl-5-dimethylamino-3-indenyl]-propionic acid.

The procedure of Examples 2A, B and C is followed using 6-dimethylamino-2-methylindanone in place of 6-methoxy-2-methylindanone and methyl -α-bromopropionate in place of methyl bromoacetate used therein. There is obtained α-[1-(ρ-methylsulfinylbenzylidene)-2-methyl-5-dimethylamino-3-indenyl]-propionic acid.

(B) Poly [α-(1-ρ-methylsulfinylbenzylidene)-2-methyl-5-dimethylamino-3-Indenyl-propionyl] chitosan

α-[1-(ρ-methylsulfinylbenzylidene)-2-methyl-5-dimethylamino-3-indenyl]-propionic acid (0.02 mol.) is conjugated to chitosan gel (0.01 mol-$NH_2$) according to Scheme I. The procedure yields the desired compound ($R_1$ = $CH_3$; $R_2$ = $CH_3$; $R_3$ = 5-dimethylamino; $R_4$ = hydrogen; $R_5$ = p-$CH_3SO$-; m > 50; n/m > 0.2; n > 10.)

EXAMPLE 6

Poly [5,6-Difluoro-2-Methyl-1-(ρ-Methylsulfinylbenzylidene)-3-Indenyl Acetyl] Polylysine

(A) 3,4-difluorobenzaldehyde.

In a 250 ml. three-necked flask equipped with a magnetic stirrer, thermometer, condenser, and dropping funnel is placed 25.6 g (0.2 mole) of 3,4 difluorotoluene. The liquid is heated to 105° and illuminated as 67 g. (0.42 mole) of bromine is added slowly. The temperature is kept between 105-110° while the first half of the bromine is added over a period of one hour. The rest of the bromine is added over approximately a 2 hour period and the temperature is raised to 150° and kept there for 5 minutes.

The reaction mixture is cooled and transferred to a 1 liter 3-necked flask with a motor driven stirrer and condenser. 120 ml. $H_20$ and 90 g. of calcium carbonate are added, and the mixture is refluxed for 20 hours with good stirring. The reaction mixture is steam distilled until no further oil is collected. The oil is taken up in methylene chloride and dried over $MgSO_4$. Evaporation of the solvent yields 3,4-difluorobenzaldehyde which is used without further purification.

(B) 3,4-difluoro-α-methylcinnamic acid.

A mixture of 2.88 g. (0.02 mole) of 3,4-difluorobenzaldehyde, 3.24 g. (0.025 mole) of propionic anhydride and 0.92 g. (0.02 mole) of sodium propionate under nitrogen is heated at 135° with a magnetic stirrer for 20 hours. The reaction mixture is poured onto 50 ml. of water. A solid precipitates that dissolves when 50 ml. of

saturated $K_2CO_3$ is added with stirring. The basic solution is extracted with ether (2 X 100 ml.). The aqueous phase is then poured into an excess of concentrated HCl and ice. The precipitated white solid is filtered and dried to give 3,4-difluoro-α-methylcinnamic acid, M.P. 122-125°.

4-TRIFLUOROMETHYL-α-METHYLCINNAMIC ACID

The above reaction of Example 6A is repeated except that 4-trifluoromethylbenzaldehyde is used as a starting material in place of 3,4-difluorobenzaldehyde. Using the same reaction conditions and techniques there is obtained 4-trifluoromethyl-α-methylcinnamic acid.

Similarly using other benzaldehydes such as 4-methylthiobenzaldehyde, 4-chlorobenzaldehyde, and 3-methyl-4-chlorobenzaldehyde, there is obtained 4-methylthio-α-methylcinnamic acid, 4-chloro-α-methylcinnamic acid and 3-methyl-4-chloro-α-methylcinnamic acid respectively.

(C) 3,4-difluoro-α-methylhydrocinnamic acid.

28 g. (0.141 mole) of 3,4-difluoro-α-methylcinnamic acid, 1 g. of $PtO_2$ in 250 ml. of MeOH is hydrogenated at 45 p.s.i. until the theoretical uptake is completed. The catalyst is filtered off, and the material evaporated to one-third its volume. A 15% potassium hydroxide solution (10 ml.) is added, and the mixture refluxed for 30 minutes when it is poured into water and extracted with ether (2 X 100 ml.). The aqueous layer is acidified with concentrated HCl and ice. The oil which comes out is extracted into ether, the ether solution dried over $MgSO_4$ and evaporated to leave a clear oil which crystallizes. 3,4-difluoro-α-methylhydrocinnamic acid, M.P. 55-56°, is isolated.

(D) 5,6-difluoro-2-methyl-1-indanone.

20 g. (0.1 mole) of 3,4-difluoro-α-methylhydrocinnamic acid is added to 250 g. of polyphosphoric acid. The mixture is efficiently stirred and heated on a steam bath for 2 hours. The mixture is poured onto ice-water (400 ml.). The precipitate is extracted with ether (3 X 100 ml.). The extract is washed with saturated potassium carbonate, water and then dried ($MgSO_4$). The ether solution, when evaporated, leaves solid 5,6-difluoro-2-methyl-1-indanone (M.P. 66-68°) which is used without further purification.

(E) 5,6-difluoro-2-methylindene-3-acetic acid methyl ester.

A mixture of 9.1 g. (0.05 mole) of 5,6-difluoro-2-methyl-1-indanone, 4.0 g. of "activated" zinc dust, 7.6 g. (0.05 mole) of methyl bromoacetate, and a crystal of iodine in 250 ml. of dry benzene is refluxed for 4-5 hours. Tlc (20% $Et_2O$-80% pet. ether on Si gel) shows greater than 95% conversion at this time. The reaction mixture is poured onto 250 ml. of 5% $H_2SO_4$, separated, and dried ($MgSO_4$). Removal of solvent leaves an oily hydroxy ester. The crude ester is redissolved in 100 ml. of benzene and phosphorus pentoxide (20 g.) is added. The mixture is refluxed for 30 minutes (no stirrer necessary) and decanted. The residue is washed with benzene, the organic layers combined, washed with water (2 X 100 ml.) and dried ($MgSO_4$). The benzene, when evaporated, leaves 5,6-difluoro-2-methylindene-3-acetic acid methyl ester, M.P. 86-90°.

5-METHYLTHIO-2-METHYLINDENE-3-ACETIC ACID METHYL ESTER

The above reaction of Example 6E is repeated using 5-methylthio-2-methylindanone instead of 5,6-difluoro-2-methyl-1-indanone. Using the same conditions and techniques, there is obtained 5-methylthio-2-methylindene-3-acetic acid methyl ester.

When an acylamino or sulfonyl indanone is employed as the starting material in the above procedure, the corresponding methyl ester is obtained.

(F) 5,6-difluoro-2-methyl-1-(ρ-methylthiobenzylidene)- indene-3-acetic acid.

1.19 g. (5.0 mmole) of 5,6-Difluoro-2-methylindene-3-acetic acid methyl ester is dissolved in 10 ml. of dry pyridine followed by 0.76 g. (5.0 mmole) of ρ-methylthiobenzaldehyde. The flask is placed under nitrogen, and 5.0 g (5.1 mmole) of Triton B is added. The deeply colored solution is allowed to stand overnight, and then water (2 ml.) is added. After standing for 15 minutes, it is poured into an excess of water. The organics are extracted with ether (2 X 50 ml.). The aqueous phase is added to 10% HCl-ice. The orange, gummy solid that precipitates is extracted into methylene chloride and dried ($MgSO_4$). The solvent is removed to leave an orange

solid. The solid is filtered to give a crude product which is recrystallized from benzene to give 5,6-difluoro-2-methyl-1-(ρ-methylthiobenzylidene)-indene-3-acetic acid. M.P. 181-182.5°.

When 3-methylthio-2-furaldehyde or 2-methylthio-5-pyrazine aldehyde is utilized in the above procedure instead of ρ-methylthiobenzaldehyde the corresponding indene acetic acid is obtained.

(G) 5,6-difluoro-2-methyl-1-(ρ-methylsulfinylbenzylidene)-indene-3-acetic acid.

To a solution of 0.358 g. (1.0 mole) of 5,6-difluoro-2-methyl-1-(ρ-methylthiobenzylidene)-indene-3-acetic acid in acetone (10 ml.) is added 10-15 ml. MeOH. With magnetic stirring, 0.32 g. (1.5 mmole) of sodium meta periodate is added in 5 ml. of water. The proportions of acetone, methanol and water are adjusted if necessary to preserve homogeneity. After several minutes, a precipitation of sodium iodate appears. The suspension is stirred at room temperature for 16 hours, and then poured into approximately 50 ml. of water and 100 ml. methylene chloride. The two phases are separated and the water layer is extracted twice with methylene chloride. The organic phases are washed with water and dried ($MgSO_4$). The residue after evaporation is dissolved in the minimum amount of boiling ethyl acetate and allowed to stand for 12 hours in the freezer compartment. The deep orange crystals are filtered. The filtrate is reduced to 1/2 volume and allowed to stand in the cold for several hours to give a large second crop. In this way, 5,6-difluoro-2-methyl-1-(ρ-methylsulfinylbenzylidene)-3-indenylacetic acid is isolated, M.P. 200-210°.

(H) Poly [5,6-difluoro-2-methyl-1-(ρ-methylsulfinylbenzylidene)-3-indenyl acetyl] polylysine

5,6-difluoro-2-methyl-1-(ρ-methylsulfinylbenzylidene)-3-indenyl acetic acid (0.01 mol.) is conjugated to polylysine (0.01 mol-$NH_2$) according to Scheme II. The procedure yields the desired compound ($R_1$ = hydrogen; $R_2$ = $CH_3$; $R_3$ = 5-fluoro; $R_4$ = 6-fluoro; $R_5$ = $CH_3$-SO-; Q = polylysine; m > 50; n/m > 0.2; n > 10).

Similarly, when 5-methylthio-2-methylindene-3-acetic acid methyl ester is used in the reactions of Examples 6(F) and 6(G) to produce 5-methylthio-2-methyl-1-(ρ-methylsulfinylbenzylidene)-3-indenyl acetic acid, the compound so produced can be conjugated to polylysine according to Example 6(H) to produce poly-5-methylthio-2-methyl-1-(ρ-methylsulfinylbenzylidene)-3-indenyl acetyl polylysine.

## EXAMPLE 7

### Poly [5,6-Difluoro-2-Methyl-1-(ρ-Methylsulfonylbenzylidene)-Indenyl-3-Acetyl] Methyl Cellulose

(A) 5-6,difluoro-2-methyl-1-(ρ-methylsulfonylbenzylidene)-indene-3-acetic acid.

To 5,6-difluoro-2-methyl-1-(ρ-methylsulfinylbenzylidene)-indene-3-acetic acid (0.005 mole) in acetone (15 ml.) is added, slowly with stirring, m-chloroperbenzoic acid (0.005 mole). The mixture is heated and evaporated to near dryness at 40°. The solid is washed with boiling water (4 X 50 ml.) and dried yielding 5,6-difluoro-2-methyl-1-(ρ-methylsulfonylbenzylidene)-indene-3-acetic acid, M.P. 228-230°.

(B) Poly [5,6-Difluoro-2-Methyl-1-(ρ-Methylsulfonylbenzylidene-Indene-3-Acetyl] Methyl Cellulose

5,6-difluoro-2-methyl-1-(ρ-methylsulfonylbenzylidene)-indene-3-acetic acid (0.01 mol.) is conjugated to methylcellulose (0.01 mol-OH) according to Scheme IV. Specifically, oxalylchloride is used to prepare the acid chloride of 5,6-difluoro-2-methyl-1-(ρ-methylsulfonylbenzylidene)-indene-3-acetic acid. Pyridine is the base. The procedure yields the desired compound ($R_1$ = hydrogen; $R_2$ = $CH_3$; $R_3$ = 5-fluoro; $R_4$ = 6-fluoro; $R_5$ = $CH_3$-$SO_2$-; Q = methylcellulose; m > 50; n/m > 0.2; n > 10).

α-[1-ρ-methylsulfonylbenzylidene)-2-methyl-5,6-difluoro-3-indenyl]-propionic acid, prepared by the procedure of Examples 3A, B and C, is similarly conjugated to methylcellulose by the procedures of Example 7B to yield poly-[α-(1-ρ-methylsulfonylbenzylidene)-2-methyl-5,6-difluoro-3-indenyl propionyl] methyl cellulose.

## EXAMPLE 8

### Poly [5,6-Difluoro-2-Methyl-1-(ρ-Methylsulfinylbenzylidene)-3-Indenyl Acetyl] Chitosan

(A) 3,4-difluorobenzaldehyde.

57 g. (0.5 mole) of ortho-difluorobenzene in 250 ml. of methylene chloride is added to 100 g. (0.75 mole)

of anhydrous aluminum chloride. The mixture is stirred (motor) and cooled in an ice bath while 85.5 g. (0.75 mole) of dichloromethyl methylether is added dropwise. Vigorous HCl evolution takes place, and the reaction mixture turns orange-red. After the addition, the mixture is stirred at room temperature for 15 minutes, and the liquid phase is decanted into 500 ml. of ice and water. The unreacted residue of aluminum chloride is washed with methylene chloride until colorless, and the washings are added to the water. The mixture is shaken well in a separation funnel until the methylene chloride layer is green. The organic layer is washed with saturated potassium carbonate solution until neutral, then dried ($MgSO_4$) and distilled to give 3,4-difluorobenzaldehyde, B.P. 70-74°/20 min. The dark residue in the distillation pot solidifies on cooling to give tris-(3-4,difluorophenyl)methane, M.P. 95-96°.

3,4-DIMETHYLBENZALDEHYDE

The above reaction of Example 6A is repeated except that o-xylene and dichloromethyl methylether are the starting materials. Using the same reaction conditions and techniques, there is obtained 3,4-dimethylbenzaldehyde.

4-MERCAPTOBENZALDEHYDE

The above reaction of Example 6A is repeated except that the starting materials are mercaptobenzene and dichloromethyl methylether. Using the same reaction conditions and techniques, there is obtained 4-mercaptobenzaldehyde.

(B) 5,6-difluoro-2-methyl-1-(ρ-methylsulfinylbenzylidene)-3-indenyl acetic acid.

The reactions of Examples 6B, 6C, 6D, 6E, 6F and 6G are repeated and 5,6-difluoro-2-methyl-1-(ρ-methylsulfinylbenzylidene)-3-indenyl acetic acid is obtained.

(C) Poly [5,6-difluoro-2-methyl-1-(ρ-methylsulfinylbenzylidene)-3-indenyl acetyl] chitosan

5,6-difluoro-2-methyl-1-(ρ-methylsulfinylbenzylidene)-3-indenyl acetic acid (0.02 mol.) is conjugated to chitosan gel (0.01 mol-$NH_2$) according to Scheme I. The procedure yields the desired compound ($R_1$ = hydrogen; $R_2$ = $CH_3$; $R_3$ = 5-fluoro; $R_4$ = 6-fluoro; $R_5$ = $CH_3SO$-; Q = chitosan; $m > 50$; $n/m > 0.2$; $n > 10$.)

Similarly, when 3,4-dimethylbenzaldehyde is used in the reactions in Example 8B, 5,6-dimethyl-2-methyl-1-(ρ-methylsulfinylbenzylidene)-3-indenyl acetic acid is obtained, which can be converted to the corresponding poly substituted chitosan conjugate according to the procedure of the Example 8(C).

When 6-mercaptobenzaldehyde is used in the reactions in Example 8B, 6-mercapto-2-methyl-1-(ρ-methylsulfinylbenzylidene)-3-indenyl acetic acid is obtained, which can be converted to the corresponding poly substituted chitosan conjugate according to the procedure of the first paragraph of Example 8(C).

## EXAMPLE 9

### Poly [α-(1-ρ-Methylsulfonylbenzylidene)-2-Methyl-5-Methoxy-6-Fluoro-3-Indenyl-Acetyl] Methylcellulose

(A) 3-fluoro-4-methoxybenzaldehyde.

To a solution of o-fluoroanisole, 101 g. (0.80 mole) in 500 ml. dry methylene chloride is added dropwise over 30 minutes a solution of titanium tetrachloride, 182 g. (0.96 mole, 1.2 equiv.) and α,α-dichloromethylmethyl ether, 110 g. (0.96 mole) in an equal volume of methylene chloride. The temperature is maintained at 10-20° C. with an ice-bath. The mixture is stirred at room temperature for 1 hour longer and then poured over crushed ice-water with stirring. Ether (1 l.) is added,and the mixture stirred under nitrogen until solution occurs. The organic layer is extracted with water (3X), sodium bicarbonate solution (3X) and dried ($MgSO_4$). The solvent is evaporated off at 30° to give crude product as an oil. The oil is vacuum distilled through a jacketed Vigreux column when it gives 3-fluoro-4-methoxybenzaldehyde, B.P. 120-121° C, at 10 mm. Hg; $R_f$ 0.6 on a silica-gel G plate with methylene chloride.

(B) 3-fluoro-4-methoxy-α-methylcinnamic acid.

A mixture of 3-fluoro-4-methoxybenzaldehyde, 34.2 g. (0.22 mole), propionic anhydride, 50 g. (0.38 mole)

and sodium propionate, 21 g. (0.22 mole) is stirred under nitrogen at 150° C for 15 hours. The reaction mixture is then poured into 1.3 l. of water with stirring, and the product is precipitated. 2.0 N potassium hydroxide solution (500 ml.) is added, and the mixture stirred for several hours, until the acid has dissolved.

The aqueous solution is extracted with ether (3X) and then acidified with concentrated hydrochloric acid with stirring. The precipitated product is collected, washed thoroughly with water and dried in a vacuum oven at 50° C. over potassium hydroxide pellets to give 3-fluoro-α-methyl-4-methoxycinnamic acid, M.P. 167-169° C.; $R_f$ 0.5 on silica-gel G with methylene chloride-methanol (1:1).

(C) 3-fluoro-4-methoxy-α-methyl dihydrocinnamic acid.

3-Fluoro-4-methoxy-α-methylcinnamic acid, (49.5 g.; 0.236 mole) in 800 ml. methanol is hydrogenated at 43 lbs. pressure and room temperature until the theoretical uptake of hydrogen has occurred (24 min at 20°C., using 1.5 g. platinum oxide catalyst). The solution is filtered and then evaporated with warming to 60° to give 3-fluoro-4-methoxy-α-methyl dihydrocinnamic acid, $R_f$ 0.5 on silica-gel G with methylene chloride-methanol (9:1).

(D) 5-fluoro-6-methoxy-2-methylindanone.

A mixture of 3-fluoro-α-methyl-4-methoxy dihydrocinnamic acid, 49.3 g. (0.23 mole) in 500 g. of polyphosphoric acid is heated at 95° C. on a steam bath with occasional agitation for 75 min. The dark red solution is poured into 3.0 liters of water, and the mixture is stirred overnight. The precipitated product is collected, washed thoroughly with water and then taken up in ether. The ether solution is extracted with aqueous potassium bicarbonate (4X), diluted with methylene chloride, and dried ($MgS0_4$).

The organic solution is evaporated and recrystallized from methylene chloride-petroleum ether to give 5-fluoro-6-methoxy-2-methylindanone, (M.P. 76-78°).

(E) Methyl 6-fluoro-5-methoxy-2-methyl-3-indenylacetate.

Into a 500 ml. three-necked flask fitted with mechanical stirrer, reflux condenser, drying tube, dropping funnel and nitrogen inlet is placed 8.0 g. zinc sheet and 100 ml. of dry benzene. A few milliliters of a solution of 21.3 g. (0.11 mole) of 5-fluoro-6-methoxy-2-methylindanone and 18.36 g. (0.121 mole) of methyl bromoacetate in 100 ml. of dry benzene is added at a time. A crystal of iodine is added. The mixture is gently heated with stirring. After the iodine color has disappeared, the remainder of the mixture is added gradually. The reaction is heated at reflux temperature for about 18 hours. The mixture is poured onto 600 ml. of 5% $H_3SO_4$ and about 500 g. of ice. Some ether is added. The organic layer is separated and washed with three portions of 5% $H_2SO_4$ water, $KHCO_3$ solution and finally water again. The organic layer is dried ($MgSO_4$) and concentrated to give 27.6 g. of reddish oil which crystallizes upon standing. Thin-layer chromatography on silica-gel G with methylene chloride methanol (99:1) shows product at $R_f$ (0.5).

Without further purification, the hydroxy ester is dehydrated to the indenylacetate. In 200 ml. of dry benzene, 14.2 g. (53 mmole) of crude ester and 36 g. of phosphorus pentoxide are refluxed with stirring for 1/2 hour. After cooling, the reaction mixture is filtered and the solid residue washed well with benzene. The benzene filtrate is washed with two portions of salt water and dried ($MgS0_4$). The organic solution is concentrated and gives a slightly colored oil which rapidly crystallizes. The crude product is recrystallized from methylene chloride-petroleum ether to give methyl-6-fluoro-5-methoxy-2-methyl-3-indenylacetate (M.P. 61-62°).

(F) 6-fluoro-5-methoxy-2-methyl-1-(ρ-methylthiobenzylidene)-3-indenyl acetic acid.

To a solution of methyl-6-fluoro-5-methoxy-2-methyl-3-indenyl acetate, 9.3 g. (0.037 mole) and ρ-methylthiobenzaldehyde, 6.3 g. (1.1 equivalent) is added 16 ml. (2.0 equivalents) of 25% methanolic sodium methoxide. The mixture is stirred at reflux under nitrogen for 2 hours. An equal volume of water is added dropwise and refluxing continues for 30 minutes. The solution is cooled, diluted with water and extracted with ether (3X). Residual ether is blown off with nitrogen, and then the aqueous solution is acidified with 50% glacial acetic acid. The precipitated product is collected and washed thoroughly with water. The crude product is recrystallized from methanol to give 6-fluoro-5-methoxy-2-methyl-1-(ρ-methylthiobenzylidene)-2-indenyl acetic acid, M.P. 172-174°.

(G) 6-fluoro-5-methoxy-2-methyl-1-(ρ-methylsulfinylbenzylidene)-3-indenyl acetic acid.

A solution of sodium periodate, 4.28 g. (20 mmole) in 40 ml. of water is added dropwise to 6-fluoro-5-methoxy-2-methyl-1-(ρ-methylthiobenzylidene)-3-indenyl acetic acid, 3.70 g. (10 mmole) in 300 ml. methanol and enough acetone to cause solution. This solution is stirred over night at room temperature and filtered. The filtrate is evaporated at 30° to a small volume which causes the product to precipitate. The suspension is diluted with several volumes of water, cooled and collected. After rinsing with water and cold methanol-water (1:1), the product is dried in vacuo over potassium hydroxide pellets, and then in a vacuum oven at 70°C. The crude product is recrystallized from methylene chloride-petroleum ether to give 6-fluoro-5-methoxy-2-methyl-1-(ρ-methylsulfinylbenzylidene)-3-indenyl acetic acid (M.P. 190-193°).

6-Fluoro-5-methoxy-2-methyl-1-(ρ-methylsulfonylbenzylidene)-3-indenyl acetic acid is prepared according to the procedure of Example 7 by the addition of 1.0 mole of m-chloroperbenzoic acid per mole of 6-fluoro-5-methoxy-2-methyl-1-(ρ-methylsulfinylbenzylidene)-3-indenyl acetic acid in an acetone solution.

α-[1-(ρ-Methylsulfonylbenzylidene)-2-methyl-5-methoxy-6-fluoro-3-indenyl]-propionic acid is prepared by the procedures of Examples 3A, 3B and 3C.

(H) Poly [α-(1-ρ-methylsulfonylbenzylidene)-2-methyl-5-methoxy-6-fluoro-3-indenyl-acetyl] methylcellulose

α-[1-(ρ-Methylsulfonylbenzylidene)-2-methyl-5-methoxy-6-fluoro-3-indenyl]-acetic acid (0.01 mol-OH) is conjugated to methylcellulose (0.01 mol.) according to Scheme III. Specifically, ethyldiisopropylamine is used as the base. The procedure yields the desired compound ($R_1$ = hydrogen; $R_2$ = $CH_3$; $R_3$ = 5-$CH_3$-O-; $R_4$ = hydrogen; $R_5$ = $CH_3$-$SO_2$-; Q = methylcellulose; m > 100; n/m > 0.2; n > 10.)

Similarly, poly-[6-fluoro-5-methoxy-2-methyl-1-(ρ-methylsulfonylbenzylidene)-3-indenyl propionyl] methyl cellulose can be produced by using 6-fluoro-5-methoxy-2-methyl-1-(ρ-methylsulfonylbenzylidene)-3-indenyl propionic acid in the procedure of the preceding paragraph.

EXAMPLE 10

Poly [α-(1-ρ-Methylsulfonylbenzylidene)-2-Methyl-5-Fluoro-3-Indenyl-Acetyl] Polyvinyl Alcohol Ester

(A) ρ-Fluoro-α-methylcinnamic acid.

ρ-Fluorobenzaldehyde (200 g., 1.61 mole), propionic anhydride (3.5 g., 2.42 mole) and sodium propionate (155 g., 1.61 mole) are mixed in a 1 l. three-necked flask which had been flushed with nitrogen. The flask is heated gradually in an oil-bath to 140°. After 20 hours, the flask is cooled to 100° and poured into 8 l. of water. The precipitate is dissolved by adding potassium hydroxide (302 g.) in 2.l of water. The aqueous solution is extracted with ether, and the ether extracts washed with potassium hydroxide solution. The combined aqueous layers are filtered, acidified with concentrated HCl, filtered and the collected solid washed with water, thereby producing ρ-fluoro-α-methylcinnamic acid which is used as obtained.

(B) ρ-Fluoro-α-methylhydrocinnamic acid.

To ρ-fluoro-α-methylcinnamic acid (177.9 g., 0.987 mole) in 3.6 l. ethanol is added 11.0 g. of 5% Pd/C and the mixture is reduced at room temperature under a hydrogen pressure of 40 p.s.i. Uptake is 31/32 lbs. (97% of theoretical). After filtering the catalyst, the filtrate is concentrated in vacuo to give the product ρ-fluoro-α-methylhydrocinnamic acid used without weighing in next step.

(C) 6-Fluoro-2-methylindanone.

To 932 g. polyphosphoric acid at 70° on the steam bath is added ρ-fluoro-α-methylhydrocinnamic acid (93.2 g., 0.5 mole) slowly with stirring. The temperature is gradually raised to 95° C., and the mixture kept at this temperature for 1 hour. The mixture is allowed to cool and added to 2 l. of water. The aqueous layer is extracted with ether, the ether solution washed twice with saturated sodium chloride solution, 5% $Na_2CO_3$ solution, water, and then dried. The ether filtrate is concentrated with 200 g. silica-gel, and added to a five pound silica-gel column packed with 5% ether-petroleum ether. The column is eluted with 5-10% ether-petroleum ether and followed by tlc to give 6-fluoro-2-methylindanone.

(D) 5-fluoro-2-methylindanone-3-acetic acid.

A mixture of 6-fluoro-2-methylindanone (18.4 g., 0.112 g. mole), cyanacetic acid (10.5 g., 0.123 mole), acetic acid (6.6 g.), and ammonium acetate (1.7 g.) in dry toluene (15.5 ml.) is refluxed with stirring for 21 hours, as the liberated water is collected in a Dean Stark trap. The toluene is concentrated, and the residue dissolved in 60 ml. of hot ethanol and 14 ml. of 2.2 N. aqueous potassium hydroxide solution. 22 g. of 8.5% KOH in 150 ml. of water is added, and the mixture refluxed for 13 hours under nitrogen. The ethanol is removed under vacuum, 500 ml. water added, the aqueous solution washed well with ether and then boiled with charcoal. The aqueous filtrate is acidified to pH 2 with 50% hydrochloric acid, cooled and the precipitate collected. In this way dried 5-fluoro-2-methylindenyl-3-acetic acid (M.P- 164-166°) is obtained.

(E) 5-fluoro-2-methyl-1-(ρ-methylthiobenzylidene)-3-indenyl acetic acid.

5-fluoro-2-methyl-3-indenyl acetic acid (15 g., 0.072 mole) ρ-methylthiobenzaldehyde (14.0 g., 0.091 mole) and sodium methoxide (13.0 g., 0.24 mole) are heated in methanol (200 ml.) at 60° under nitrogen with stirring for 6 hours. After cooling, the reaction mixture is poured into 750 ml. of ice-water, acidified with 2.5 N hydrochloric acid, and the collected solid triturated with a little ether to produce 5-fluoro-2-methyl-1-(ρ-methylthiobenzylidene)-3-indenyl acetic acid (M-P. 187-188.2°). U.V. in methanol $\lambda_{max}$. 348 mμ (E% 500), 258 (557), 258 (495), 353 (513), 262.5 (577), 242.5 (511).

(F) 5-fluoro-2-methyl-1-(ρ-methylsulfinylbenzylidene)-3-indenyl acetic acid.

To a solution of 5-fluoro-2-methyl-1-(ρ-methylthiobenzylidene)-3-indenyl acetic acid (3.4 g., 0.01 mole) in a mixture of methanol (250 ml.) and acetone (100 ml.) is added a solution of sodium periodate (3.8 g., 0.018 mole) in water (50 ml.) with stirring.

Water (450 ml.) is added after 18 hours, and the organic solvents removed under vacuum below 30°. The precipitated product is filtered, dried and recrystallized from ethyl acetate to give 5-fluoro-2-methyl-1-(ρ-methylsulfinylbenzylidene)-3-indenyl acetic acid. Upon repeated recrystallization upon ethylacetate there is obtained cis-5-fluoro-2-methyl-1-(ρ-methylsulfinylbenzylidene)-3-indenyl acetic acid, M.P. 184-186°. U.V. in methanol; $\lambda_{max}$ 328 (E% 377), 286 (432), 257.5 shldr. (413), 227 (548).

Further runs reveal the existence of a second polymorph of cis-5-fluoro-2-methyl-1-(ρ-methylsulfinylbenzylidene)-3-indenyl acetic acid, M.P. 179-181° C.

5-chloro-2-methyl-1-(ρ-methylsulfinylbenzylidene)-3-indenyl acetic acid is prepared by the procedure as described in Example 10.

(G) 5-fluoro-2-methyl-1-(p-methylsulfonylbenzylidene)-3-indenyl acetic acid.

Sodium methoxide (4.4 M in methanol, 68.5 ml, 0.3 mol) was added dropwise to a stirred cooled mixture of 5-fluoro-2-methyl-1-(ρ-methylsulfinylbenzylidene)-3-indenyl acetic acid (100 g, 0.281 mol) in methanol (250 ml) and acetonitrile (500 ml). Sodium bicarbonate (0.56 mol) and hydrogen peroxide (30% in water, 0.56 mol) were added and allowed to react for 18 hours at -10°C. Excess sodium bicarbonate was filtered off, and cooled filtrate (0°C) neutralized dropwise to pH 7 with 1 M hydrochloric acid (350 ml). The resulting product was then filtered and washed with methanol. A thin layer chromatography system to check for purity utilizes chloroform:methyl isobutyl ketone (8:2); the $R_f$ value is 0.21. A tetrahydrofuran/diisopropyl ether combination was used for product recrystallization. Reaction yield is 89%. The product has a mp of 205-206°C.

α-[1-(ρ-methylsulfonylbenzylidene)-2-methyl-5-fluoro-3-indenyl]-propionic acid are prepared by the procedures of Examples 3A, 3B and 3C.

(H) [α-(1-p-methylsulfonylbenzylidene)-2-methyl-5-fluoro-3-indenyl)-Acetic Acid Chloride

Thionyl chloride (2.00 ml) was added to a suspended solution of 5 g (0.0134 mol) of [α-(1-p-methylsulfonylbenzylidene)-2-methyl-5-fluoro-3-indenyl)-acetic acid in 30 ml toluene. The mixture was refluxed under nitrogen for 60 minutes. The solvent was removed in vacuo to give [α-(1-p-methylsulfonylbenzylidene)-2-methyl-5-fluoro-3-indenyl)-acetic acid chloride as a red oil. Purification by crystallization in a solvent mixture of toluene/petroleum ether gave 4.90 g of [α-(1-p-methylsulfonylbenzylidene)-2-methyl-5-fluoro-3-indenyl)-acetic acid chloride as a solid. IR(Nujor Mull) 2948, 2912, 1795, 1605, 1460, 1402, 1377, 1305, 1156 $cm^{-1}$.

(I) Poly [α-(1-p-methylsulfonylbenzylidene)-2-methyl-5-fluoro-3-indenyl)-Acetyl] polyvinyl alcohol ester

[α-(1-p-methylsulfonylbenzylidene)-2-methyl-5-fluoro-3-indenyl)-acetic acid chloride 4.9 g (0.013 mol) was dissolved in 100 ml of pyridine. To this solution was added 1.1 g (0.031 mol) of polyvinyl alcohol ("PVA", average molecular weight of 50,000). The reaction mixture was slowly heated until all PVA dissolved and then refluxed for 60 minutes. The clear solution was concentrated in vacuo then washed with 100 ml of concentrated sodium bicarbonate solution. The aqueous part was decanted and the residual oil was washed with five 200 ml portions of water to remove pyridine and unreacted PVA. The product was dried over phosphorus pentoxide ($P_2O_5$) in a vacuum desiccator at room temperature for 12 days. It gave 3.5 g of Poly [α-(1-p-methylsulfonylbenzylidene)-2-methyl-5-fluoro-3-indenyl)-Acetyl] polyvinyl alcohol ester as a solid.

IR (Nujor mull) 3442, 3061, 2927, 2889, 1718, 1625, 1607, 1467, 1377, 1307 $cm^{-1}$.

Elemental analysis calculated for $C_{24}H_{23}FO_5S$, FW 442; % Theory: C, 65.19; H, 5.20; S, 7.23; F, 4.29. % Found : C, 65.29; H, 4.69; S, 8.05; F, 4.67.

The procedure yields the desired compound $R_1$ = hydrogen; $R_2$ = methyl; $R_3$ = 5-fluoro; $R_4$ = hydrogen; $R_5$ = $CH_3SO_2$; Q = polyvinyl alcohol; n/m = .50.

In addition, poly-5-fluoro-2-methyl-1-(ρ-methylsulfonylbenzylidene)-3-indenyl propionyl polyvinyl alcohol ester can be produced using the propionic acid from part (F) of this example in the process of parts (H) and (I) of this Example.

## EXAMPLE 11

### Poly [Cis-5,7-Difluoro-2-Methyl-1-(ρ-Methylsulfinylbenzylidene)-3-Indenyl Acetyl]Chitosan

(A) 2,4-difluorobenzaldehyde.

A 250 ml., three-necked flask is fitted with a stirrer, a thermometer, a dropping funnel with a long stem to the bottom of the flask and a reflux condenser with a tube leading to the back of a hood. 50 g. (0.38 mole) of 2,4-difluorotoluene is heated to reflux with stirring and irradiated with a Hanovia ultraviolet lamp. 41.5 ml. of bromine is gradually added. The reaction is completed in 2.5 hours during which time the reflux temperature rises from 112° to 155°.

A 2 l. three-necked flask is fitted with a stirrer and reflux condenser. In the flask is placed 200 ml. of water and 140 g. calcium carbonate. The cooled above-described reaction mixture is transferred using some ether for rinsing. The hydrolysis is completed by refluxing with stirring for 18 hours. The aldehyde is isolated by steam distillation from the reaction flask. The oil is separated and the aqueous phase is extracted once with ether. The combined oil and ether extract is dried over anhydrous $MgSO_4$ and concentrated under reduced pressure to leave 2,4-difluorobenzaldehyde, still containing some ether which is distilled through a short Vigreux column under reduced pressure and separated into several fractions. These are combined to give 2,4-difluorobenzaldehyde, B.P. 56-58° 12 mm.

(B) 2,4-difluoro-α-methylcinnamic acid.

A 500 ml., three-necked flask is fitted with reflux condenser, drying tube, stirrer and $N_2$ inlet. To a mixture of 55.4 g. (0.39 mole) of 2,4-difluorobenzaldehyde and 56 ml. of propionic anhydride is added 38 g. (0.39 mole) of sodium propionate. The reaction mixture is heated at 135-140° (oil bath temp.) for 19 hours with stirring under nitrogen. The still warm solution is poured into 1 l. of water with stirring. A solid separates, which upon adding 56 g. of potassium hydroxide, dissolves. The solution is extracted with ether, and then heated on the steam bath to remove the ether. After cooling in an ice-bath, concentrated hydrochloric acid is added with stirring. The product which separates is collected and washed with cold water. After drying at 60°C over KOH, 2,4-difluoro-α-methylcinnamic acid, M.P. 126-128° is obtained.

(C) 2,4-difluoro-α-methylcinnamic acid.

In 800 ml. of methanol, 60 g. (0.3 mole) of 2,4-difluoro-α-methylcinnamic acid with 1.5 g. of platinum oxide catalyst is shaken under an initial pressure of 42 lbs. of hydrogen until one equivalent of hydrogen is absorbed. The reaction time is 30 minutes. The catalyst is removed by filtration and washed with methanol. The methanol, when evaporated off, leaves near colorless 2,4-difluoro-α-methyldihydrocinnamic acid as an oil which is used in the next step without further purification.

(D) 4,6-difluoro-2-methylindanone.

A solution of 2,4-difluoro-α-methyldihydrocinnamic acid, 54.8 g. (0.274 mole) in 125 ml. thionyl chloride is stirred for 90 minutes, and then at reflux for 90 minutes longer. The reaction solution is evaporated under reduced pressure leaving the acid chloride product as an oil.

To a suspension of ice-bath cooled anhydrous powdered aluminum chloride, 60 g. (0.45 mole), in 250 ml. of dry carbon disulfide is added dropwise over 10 minutes, a solution of the acid chloride, 60 g., in 100 ml. carbon disulfide. After the addition, the ice bath is removed, and the temperature raised slowly to room temperature. The mixture is stirred at room temperature for 20 hours, and then is poured into 2 l. of 10 aqueous hydrochloric acid-crushed ice with stirring. Ether is added, and the stirring continued until everything dissolves. The ether layer is extracted with 5% hydrochloric acid (2X), water (2X), and sodium bicarbonate solution (2X), when it is diluted with methylene chloride and dried ($MgSO_4$). The filtered solution is evaporated with warming to 70° C. to give the crude 4,6-difluoro-α-methylindanone as an oil which crystallizes on standing. The crude product is purified by chromatography of a column (7.0 X 35 cm.) of silicagel, 400 g. of J.T. Baker 3405 packed in petroleum ethermethylene chloride (2:1). The column is developed and eluted with the same solvent system, and upon recrystallization from methylene chloride-petroleum ether gives 4,6-difluoro-2-methylindanone, M.P. 68-69° C.

(E) Methyl 5,7-difluoro-2-methylindenyl -3-acetate.

About 20% of a solution containing 4,6-difluoro-2-methylindanone, 15.0 g. (83 mmole), and methyl bromoacetate, 14.0 g. (1.1 equiv.) in 100 ml. dry benzene is added to a stirred suspension of powdered zinc dust (merck dried 120°/22 mm.), 6.5 g. (1.2 equiv.) in 74 ml. dry benzene under a nitrogen atmosphere. Several crystals of iodine are added, and the mixture slowly brought to a reflux. The remainder of the solution is added dropwise over 10 minutes, and the mixture stirred at reflux overnight, i.e., 17 hours. The reaction is cooled to room temperature, the mixture poured into 2.0 l. of 20% aqueous sulfuric acid-crushed ice with stirring, and ether added until a clear solution is obtained. The ether layer is extracted with 5% aqueous sulfuric acid (3X), water (3X), diluted with methylene chloride and dried ($MgSO_4$). The filtered etheral solution is evaporated to give crude hydroxy ester.

Powdered phosphorus pentoxide (60.0 g.) is added to the hydroxy ester (20.0 g.) in 400 ml. of dry benzene. The mixture is stirred at reflux for 30 minutes, and the clear benzene solution decanted. The residue is rinsed with benzene and then with ether. The combined organic solutions are diluted with ether, extracted six times with aqueous sodium sulfate solution, twice with aqueous potassium bicarbonate solution, diluted with methylene chloride and dried ($MgSO_4$). The crude indenyl acetate product is obtained by evaporation of the filtered elution to give an oil. The product is crystallized from petroleum ether and gives methyl 5,7-difluoro-2-methylindenyl-3-acetate, M.P. 69-70° C.

(F) 5,7 difluoro-2-methyl-1-(ρ-methylthiobenzylidene)-3-indenyl acetic acid, a mixture of geometric isomers.

Powdered sodium methoxide, 2.2 g. (40 mmole) is added to a suspension of methyl 5,7-difluoro-2-methylindenyl-3-acetate (4.78 g.) (20 mmole) and ρ-methylthiobenzaldehyde, 3.35 g. (22 mmole), in 40 ml. dry methanol under nitrogen. A clear solution results which is refluxed for 60 minutes. An equal volume of water is added, and refluxing continued under nitrogen for 30 minutes to complete saponification. The solution is diluted with several volumes of water and extracted with ether. Nitrogen is bubbled through the aqueous solution to remove the residual ether solvent. Fifty percent aqueous acetic acid (40 ml.) is used to precipitate the product. The product is collected and washed well with water. Then it is dried in a desiccator over potassium hydroxide pellets, and finally in the oven at 100°. The crude product is recrystallized from methylene chloride-petroleum ether and gives a mixture of the cis and trans isomers of the acid, M.P. 164-173° in a 1:3 ratio, identifiable by integrating the 2-$CH_3$ signal in the N.M.R. spectra at 7.82γ for cis and 8.20γ for trans.

(G) Cis-methyl-5,7-difluoro-2-methyl-1-(ρ-methylthiobenzylidene)-3-indenylacetate isolation by column chromatography.

Four drops of concentrated sulfuric acid are added to a solution of 5,7-difluoro-2-methyl-1-(ρ-methylthiobenzylidene)-3-indenyl acetic acid, 1.0 g. (2.8 mmole) in 60 ml. of dry methanol, and the solution stirred at reflux overnight. The solution is cooled and crystals separated which are collected, rinsed with cold methanol-water (1:1) and dried over potassium hydroxide pellets. These crystals are found to be about 95% of the trans-isomer, and could be further purified by recrystallizing from methanol giving the trans-isomer, M.P. 106-106.5°

C. Powdered potassium bicarbonate is added to the filtrate from the first crop of crystals, followed by water. A second crop of mixed ester is obtained in this way which is cis-enriched and used for chromatography.

1.7 g. of cis and trans-mixed esters are chromatographed on a column (3.0 X 90 cm.) of silica-gel, 250 g- of J.T. Baker 3405, packed in methylene chloride-petroleum ether (1:9). The column is developed and eluted with a 1:4 ratio of the same solvents. 0.3 to 0.4 l. cuts are taken as the yellow bands are eluted. In this way the trans-isomer and the cis-isomer (M.P. 94-94°) are obtained; U.V. of trans in $MeOH_{max}$ 217mμ., 256 and 362 mμ; U.V. of cis-isomer in MeOH $\lambda max_{218}$ mμ., 260 and 357 mμ.

(H) Cis-5,7-difluoro-2-methyl-1-(ρ-methylthiobenzylidene)-3-indenyl acetic acid.

1.0 N aqueous sodium hydroxide 3.0 ml. (3.0 mmole) is added to cis-methyl 5,7-difluoro-2-methyl-1-(ρ-methylthiobenzylidene)-3-indenylacetate, 250 mg. (0.64 mmole) in 20 ml. methanol under nitrogen. The mixture is refluxed for 1 hour, cooled, diluted with water and acidified with several ml. of 50% acetic acid. Crystals form and after further chilling in ice bath, they are collected, worked thoroughly with water and sucked nearly dry. The product is recrystallized from methanol-water, dried over potassium hydroxide pellets in a vacuum desiccator and finally in a vacuum oven at 100°. In this way cis-5,7-difluoro-2-methyl-1-(ρ-methylthiobenzylidene)-3-indenyl acetic acid (M.P. 182-184°) is obtained.

(I) Cis-5,7-difluoro-2-methyl-1-(ρ-methyl sulfinylbenzylidene)-3-indenyl acetic acid.

Sodium periodate 214 mg. (1.0 mmole) in 2 ml. water is added to cis-5,7-difluoro-2-methyl-1-(ρ-methylthiobenzylidene)-3-indenyl acetic acid, 170 mg. (0.475 mmole) in 12 ml. of methanol and about 0.5 ml. acetone at room temperature. The mixture is stirred overnight when inspection of tlc on silica-gel G using methylene chloride-methanol elution (1:1) shows that there is no starting material present but a trace of sulfone at $R_f$ 0.55. The reaction mixture is filtered and concentrated to a small volume without heating and diluted with water. The product is collected, rinsed with water and dried over potassium hydroxide pellets in a vacuum desiccator and finally in the oven desiccator at 80°. The product is recrystallized from ethyl acetate-petroleum ether and gives pure cis-5,7-difluoro-2-methyl-1-(ρ-methylsulfinylbenzylidene)-3-indenyl acetic acid, M.P. 188-189° C.

(J) Poly [Cis-5,7-difluoro-2-methyl-1-(ρ-methylsulfinylbenzylidene)-3-indenyl-acetyl] chitosan

Cis-5,7-difluoro-2-methyl-1-(ρ-methylsulfinylbenzylidene)-3-indenyl acetic acid (0.02 mol.) is conjugated to chitosan gel (0.01 mol-$NH_2$) according to Scheme I. The procedure yields the desired compound ($R_1$ = hydrogen; $R_2$ = $CH_3$; $R_3$ = 5-fluoro; $R_4$ = 7-fluoro; $R_5$ = $CH_3SO$; Q = chitosan; m > 50; n/m > 0.2; n > 10.)

EXAMPLE 12

Poly-[α-(1-Methylsulfinylbenzylidene-2-methyl-5,6-Difluoro-3-Indenyl)-Propionyl] Polylysine

α[1-ρ-methylsulfinylbenzylidene)-2-methyl-5,6-difluoro-3-indenyl]-propionic acid (0.01 mol.) prepared by the procedure of Example 3A, B and C is conjugated to polylysine (0.01 mol-$NH_2$) according to Scheme II. The procedure yields the desired compound ($R_1$ = hydrogen; $R_2$ = $CH_3$; $R_3$ = 5-fluoro; $R_4$ = 6-fluoro; $R_5$ = $CH_3SO$; Q = polylysine; m > 50; n/m > 0.2; n > 10).

EXAMPLE 13

Poly-[α-(1-ρ-Methylsulfinylbenzylidene-2-Methyl-5-Fluoro-6-Methoxy-3-Indenyl)-Propionyl] Polylysine

α[1-ρ-methylsulfinylbenzylidene)-2-methyl-5-fluoro-6-methoxy-3-indeny]-propionic acid is prepared by the procedures of Examples 3A-3C, and conjugated to polylysine according to Scheme II to produce the title compound ($R_1$ = methyl; $R_2$= methyl; $R_3$ = 5-fluoro; $R_4$ = 6-methoxy; $R_5$ = $CH_3$-SO; Q = polylysine; m > 50; n/m > 0.2; n> 10).

EXAMPLE 14

Poly-[α-(1-ρ-Methylsulfinylbenzylidene-2-Methyl-5-Fluoro-3-Indenyl)-Propionyl] Polylysine

α[1-(ρ-methylsulfinylbenzylidene)-2-methyl-5-fluoro-3-indenyl propionic acid is prepared by the procedures of Examples 3A-3C, and conjugated to polylysine according to Scheme II to produce the title compound ($R_1$ = methyl; $R_2$ = methyl; $R_3$ = 5-fluoro; $R_4$ = hydrogen; $R_5$ = $CH_3$-SO; Q = polylysine; m > 50; n/m > 0.2; n > 10).

EXAMPLE 15

Poly [5-fluoro-2-methyl-1-(p-methylsulfonylbenzylidene)-3-indenylacyl] chitosan

5-fluoro-2-methyl-1-(p-methylsulfonylbenzylidene)-3-indenyl acetic acid was prepared by the procedures of Example 10A-10G, and conjugated to chitosan by the following procedure.

Chitosan gel (10 g) was dissolved in water (40 ml) and adjusted to pH 3 with concentrated hydrochloric acid. Dimethylacetamide (80 ml) was then added, and the mixture was homogenized with a vibromixer for two hours to give solubilized chitosan.

5-fluoro-2-methyl-1-(p-methylsulfonylbenzylidene)-3-indenyl acetic acid (1.49 g, 4 mmol) in tetrahydrofuran (THF; 30 ml) was added to to 1-(3-dimethylaminopropyl) propyl carbodiimide (0.83 ml, 4.2 mmol) in THF (5 ml) at 0°C. Solubilized chitosan (40 ml) and diisopropyl ethylamine (0.8 ml) were then added, and the entire mixture stirred for 72 hours. Following concentration in vacuo, sodium hydroxide (3 M; 1 ml) was added, and the mixture dialyzed against water (24 hours). The residual content of the dialysis was centrifuged, and the precipitate and decant were both lyophilized to yield 0.20 g and 1.10 g, respectively, of the poly [5-fluoro-2-methyl-1-(p-methylsulfinylbenzylidene)-3-indenylacy] chitosan.

IR (KBr): 3420, 2920, 2880, 1650, 1600, 1470, 1210, 1080 $cm^{-1}$

$^1H$ NMR: 7.86 - 8.04 (q), 6.70 - 7.18 (m), 3.40 (br), 3.30 (s), 2.10 (s)

The procedure yields the desired compound $R_1$ = hydrogen; $R_2$ = methyl; $R_3$ = 5-fluoro; $R_4$ = hydrogen; $R_5$ = $CH_3SO_2^-$; Q = chitosan; n/m = .05.

EXAMPLE 16

Poly [5-fluoro-2-methyl-1-(p-methylsulfinylbenzylidene)-3-indenylacyl] chitosan

5-fluoro-2-methyl-1-(p-methylsulfinylbenzylidene)-3-indenyl acetic acid was prepared by the procedures of Example 10A-10F, and conjugated to chitosan by the following procedure.

Chitosan gel (10 g) was dissolved in water (40 ml) and adjusted to pH 3 with concentrated hydrochloric acid. Dimethylacetamide (80 ml) was then added, and the mixture was homogenized with a vibromixer for two hours to give solubilized chitosan.

5-fluoro-2-methyl-1-(p-methylsulfinylbenzylidene)-3-indenyl acetic acid (1.49 g, 4 mmol) in tetrahydrofuran (THF; 30 ml) was added to to 1-(3-dimethylaminopropyl) propyl carbodiimide (0.63 ml, 3.2 mmol) in THF (5 ml) at 0°C. Solubilized chitosan (40 ml) and diisopropyl ethylamine (0.8 ml) were then added, and the entire mixture stirred for 72 hours. Following concentration in vacuo, sodium hydroxide (3 M; 1 ml) was added, and the mixture dialyzed exhaustively against water. After 24 hours, the residual content of the dialysis was centrifuged. The precipitate and decant were both lyophilyzed to yield 0.13 g and 0.90 g, respectively, of the poly [5-fluoro-2-methyl-1-(p-methylsulfinylbenzylidene)-3-indenylacyl] chitosan.

IR (KBr): 3400, 3200, 2920, 1640, 1470, 1350, 1200, 1090 $cm^{-1}$

$^1H$ NMR: 7.68 - 7.79 (q), 7.01 - 7.20 (m), 3.35 (br), 3.18 (s), 2.81 (s), 2.10 (s)

The procedure yields the desired compound $R_1$ = hydrogen; $R_2$ = methyl; $R_3$ = 5-fluoro; $R_4$ = hydrogen; $R_5$ = $CH_3SO$; Q = chitosan; n/m = .06.

EXAMPLE 17

Poly [5-fluoro-2-methyl-1-(p-methylsulfinylbenzylidene)-3-indenylacyl] ethyleneimine

5-fluoro-2-methyl-1-(p-met hylsulfinylbenzylidene)-3- indenyl acetic acid was prepared by the procedures of Example 10A-10F, and conjugated to polyethyleneimine by the following procedure.

5-fluoro-2-methyl-1-(p-methylsulfinylbenzylidene)-3-indenyl acetic acid (0.72 g, 2 mmol) in tetrahydrofuran (25 ml) was added to a cold solution (0°C) of 1-(3-dimethylaminopropyl) ethyl carbodiimide hydrochloride (0.42 g, 2.1 mmol) in tetrahydrofuran (10 ml). After one hour, polyethyleneimine (0.52 ml, 33% aq. solution) was added, and the reaction mixture stirred for 20 hours. methanol (40 ml) was then added, and the resulting mixture concentrated in vacuo and then dialyzed against methanol/water (9:1; 48 hours). The solution was then concentrated in vacuo, frozen and lyophilized to yield a yellow powder (0.25 g).

IR (KBr): 3450, 2950, 1600, 1460, 1050 $cm^{-1}$.

$^1H$ NMR: 7.40 - 7.81 (m), 6.70 - 7.10 (m), 2.75 (d), 2.00 - 2.28 (m).

The procedure yields the desired compound $R_1$ = hydrogen; $R_2$ = methyl; $R_3$ = 5-fluoro; $R_4$ = hydrogen; $R_5$ = $CH_3SO$; Q = polyethyleneimine; n/m = .14.

## EXAMPLE 18

Poly [5-fluoro-2-methyl-1-(p-methylthiobenzylidene)-3-indenylacyl] ethyleneimine

(A) 6-fluoro-2-methyl-3-(p-methylthiobenzyl)indene

Twenty-five grams (1.04 mol) of magnesium turnings, a crystal of iodine, and 2 ml of p-methylthiobenzyl chloride were stirred in 400 ml of anhydrous ether under nitrogen at reflux until Grignard formation commenced. A total of 39.7 g (0.23 mol, including the initial 2 ml) of p-methylthiobenzyl chloride was added dropwise so as to maintain a gentle reflux. Refluxing was continued for 15 min. A 25-30% solution of 5-fluoro-2-methyl-1-indanone in toluene was added dropwise over 50 min at 25-35°C. Over 15 minutes, the reaction mixture was quenched with 120 ml of 3 N sulfuric acid at 30-35°C. The aqueous layer was discarded and the organic layer was stirred vigorously for 1 hour with 80 ml of 1:10 concentrated sulfuric acetic acid. This was washed with 2 X 100 ml of water, 200 ml of 2 N sodium hydroxide and again with water. The organic layer was concentrated under vacuum to yield 6-fluoro-2-methyl-3-(p-methylthiobenzyl)indene as an oil.

(B) 6-fluoro-2-methyl-3-(p-methylthiobenzyl)-1-indenyl-indeneacetic acid

Crude 6-fluoro-2-methyl-3-(p-methylthiobenzyl)indene oil (11.1 g; 0.039 mol) was placed under nitrogen, warmed to 42°C, and to it was added 42.6 ml of 37.8% benzyltrimethylammonium hydroxide in methanol. To this stirred two-phase mixture at 37°C was added 6.56 ml (4.34 g on a dry basis, 0.0586 mol) of 50% glyoxylic acid in water. For 45 min the reaction mixture was stirred at 50°C. It was transferred hot to a stirred, jacketed, three-necked separatory flask and diluted with water (67 ml) and toluene (100 ml). The pH was adjusted to 2.0-2.2 with sulfuric acid. With steam on the jacket, the mixture was stirred at 70-75°C until complete solution was obtained. The water layer was discarded, and the washing repeated with hot water. One percent ammonium chloride (67 ml) was added and the pH adjusted to 8.5 with ammonium hydroxide (15 ml). The toluene layer was extracted again at pH 8.5 with hot 1% ammonium chloride. To the combined aqueous extracts was added 1,2-dichloroethane (105 ml), and the temperature adjusted to 65-68°C. The pH was adjusted to 2.0 with concentrated hydrochloric acid, and the mixture stirred at 70°C for 1 hour. The organic layer was separated, cooled, filtered, and washed to give 10 g (75%) of orange product, mp 182-184°C .

UV max 261 nm (0.1 N hydrochloric acid in methanol; ε 40 500); $^1$NMR ($Me_2SO{\bullet}d_6$), 2.1 (s, 3, $C_2$ $CH_3$), 2.4 (s, 3, $SCH_3$), 3.9 (s, 2 benzyl $CH_2$), 6.5 (s, 1, vinyl H), 7.0-7.4 (m, 6, aromatic H), 8.2 and 8.4 (d, 1, aromatic $C_7$ H). Analysis calculated for $C_{20}H_{17}FO_2S$: C, 70.57; H, 5.03; F, 5.58. Found: C, 70.621; H, 4.82; F, 5.48.

(C) 5-fluoro-2-methyl-1-(p-methylthiobenzylidene)-3-indenylacetic acid

A solution of glacial acetic acid (200 ml), concentrated hydrochloric acid (60 ml), and water (20 ml) was prepared. There was added 19g (0.056 mol) of 6-fluoro-2-methyl-3-(p-methylthiobenzyl)-1-indenylindeneacetic acid, and the slurry was heated at 90-95°C for 10 hours during which the suspended solids changed from orange to yellow. The cooled mixture was filtered, washed with water, and vacuum dried at 80-90°C to give 17.7 g (93%) of the desired product; mp 180-184°C. Purification was achieved by digesting 7.5 g in 38 ml of refluxing 1,2-dichloroethane for three hours. The mixture was cooled, filtered and washed with fresh solvent: mp 189.5-192.5°C; UV max 350 nm (ε 17 500) and 258 (19 400) in 0.1 N hydrochloric acid in methanol; $^1$NMR ($Me_2SO{\bullet}d_6$), 2.2 (s, 3, $C_2$ $CH_3$), 2.6 (s, 3, $SCH_3$), 3.6 (s, 2, $C_3$ $CH_2CO_2$), 6.6-7.7 (m, 8, aromatic vinyl H). Analysis calculated for $C_{20}H_{17}FO_2S$: C, 70.57; H, 5.03; F, 5.58. Found: C, 70.70; H, 5.02; F, 5.36.

(D) Poly [5-fluoro-2-methyl-1-(p-methylthiobenzylidene)-3-indenylacyl] ethyleneimine

5-fluoro-2-methyl-1-(p-methylthiobenzylidene)-3-indenyl acetic acid (1.0 g) in tetrahydrofuran (25 ml) was added to a cold solution (0°C) of 1-(3-dimethylaminopropyl) ethyl carbodiimide hydrochloride (0.4 2 g, 2.1 mmol) in tetrahydrofuran (10 ml). After one hour, polyethyleneimine (0.52 ml, 33% aq. solution) was added and the reaction mixture stirred for 20 hours. Methanol (40 ml) was then added, and the resulting mixture concentrated _in vacuo_ and then dialyzed against methanol/water (9:1; 48 hours). The solution was then concentrated _in_

vacuo, frozen and lyophilized to yield the desired product.

The procedure yields the desired compound $R_1$ = hydrogen; $R_2$ = methyl; $R_3$ = 5-fluoro; $R_4$ = hydrogen; $R_5$ = $CH_3S$; Q = polyethyleneimine; n/m > 0.2.

EXAMPLE 19

Poly [5-fluoro-2-methyl-1-(p-methylsulfinylbenzylidene)-3-indenylacyl] allylamine

5-fluoro-2-methyl-1-(p-methylsulfinylbenzylidene)-3-indenyl acetic acid was prepared by the procedures of Example 10A-10F, and conjugated to polyallylamine by the following procedure.

5-fluoro-2-methyl-1-(p-methylsulfinylbenzylidene)-3-indenyl acetic acid (0.36 g, 1 mmol) in tetrahydrofuran (25 ml) was added to a cold solution (0°C) of 1-(3-dimethylaminopropyl) ethyl carbodiimide (0.22 g, 1.1 mmol) in tetrahydrofuran (10 ml). After one hour, polyallylamine hydrochloride (0.22 g) in water (2 ml) was added, and the reaction mixture stirred for 43 hours. Water (40 ml) was then added, and the resulting mixture concentrated in vacuo, and then dialyzed (24 hours against tetrahydrofuran followed by 24 hours against water). The solution was then concentrated in vacuo, frozen and lyophilized to yield a yellow powder (0.08 g).

IR (KBr): 3480, 2960, 1600, 1360, 1080 $cm^{-1}$.

$^1H$ NMR: 7.63 - 7-74 (m), 6.50 - 7.13 (m), 4.76 (s), 3.58 (s), 2.82 (s), 2.2 (s), 1.25 (s).

The procedure yields the desired compound $R_1$ = hydrogen; $R_2$ = methyl; $R_3$ = 5-fluoro; $R_4$ = hydrogen; $R_5$ = $CH_3SO$; Q = polyallylamine.

It will be understood that various changes and modifications can be made in the details of procedure, formulation and use without departing from the spirit of the invention, especially as defined in the following claims.

## Claims

1. A compound of the formula:

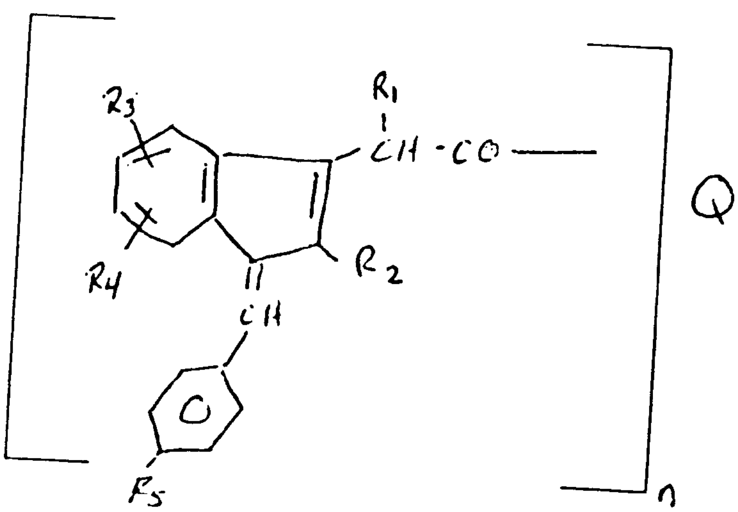

n is an integer of at least 2;

Q is a deprotonated residue of polymer or macromolecular structure having a molecular weight of at least about 1000 containing at least two primary and/or secondary amino groups and/or hydroxy groups;

$R_1$ is selected from hydrogen, lower alkyl, or haloalkyl;

$R_2$ is selected from hydrogen or alkyl;

$R_3$ and $R_4$ are one or more members each independently chosen from hydrogen, alkyl, acyloxy, alkoxy, nitro, amino, acylamino, alkylamino, diakylamino, dialkylaminoalkyl, sulfamyl, alkythio, mercapto, hydroxy, hydroxyalkyl, alkylsulfonyl, halogen, cyano, carboxyl, carbalkoxy, carbamido, haloalkyl or cycloalkoxy; and

$R_5$ is selected from alkylsulfenyl, alkylsulfinyl or alkylsulfonyl.

2. A compound as claimed in claim 1 wherein $R_3$ and $R_4$ are selected from alkyl, acyloxy, alkoxy, halogen,

haloalkyl or cycloalkoxy.

3. A compound as claimed in claim 2 wherein $R_1$ is hydrogen.

4. A compound as claimed in any preceding claim wherein n is at least 40.

5. A compound as claim in any preceding claim wherein Q contains the deprotonated residue of primary amino groups.

6. A compound as claimed in any one of claims 1 to 4 wherein Q is selected from chitosan, polyamino acids, and aminoethylcellulose.

7. A compound as claimed in claim 6 wherein said polyaminoacids contain epsilon amino groups.

8. A compound as claimed in any one of claims 1 to 4 wherein Q is a deprotonated residue of a polyhydroxy polymer.

9. A compound as claimed in claim 8 wherein Q is a deprotonated residue of polyvinylalcohol or of polymeric carbohydrate.

10. A pharmaceutical preparation or composition comprising a compound as claimed in any one of the preceding claims and a pharmaceutically acceptable carrier.

11. Use of a compound as claimed in any one of claims 1 to 9 in the manufacture of a pharmaceutical preparation or composition for the treatment or prevention of colonic polyps.

**Claims for the following Contracting State: ES**

1. A method of manufacturing a pharmaceutical composition for the treatment of colonic polyps comprising conjugating a compound of formula I

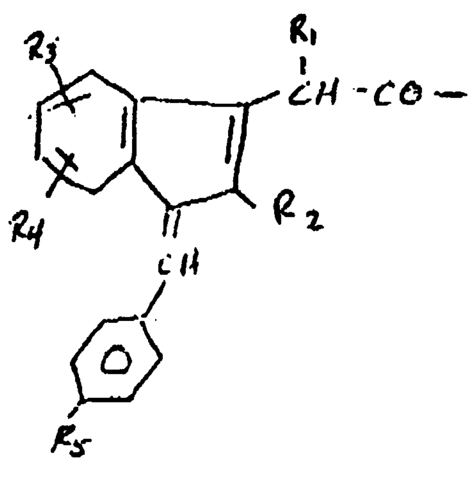

wherein $R_1$ is selected from the group consisting of hydrogen, lower alkyl, or haloalkyl;

$R_2$ is selected from the group consisting of hydrogen or alkyl;

$R_3$ and $R_4$ are one or more members each independently chosen from the group consisting of hydrogen, alkyl, acyloxy, alkoxy, nitro, amino, acylamino, alkylamino, diakylamino, dialkylaminoalkyl, sulfamyl, alkythio, mercapto, hydroxy, hydroxyalkyl, alkysulfonyl, halogen, cyano, carboxyl, carbalkoxy, carbamino, haloalkyl or cycloalkoxy; and

$R_5$ is selected from the group consisting of alkylsulfenyl, alkylsulfinyl or alkylsulfonyl;

with a deprotonated residue of polymer or macromolecular structure having a molecular weight of at least about 1000 containing at least two primary and/or secondary amino groups and/or hydroxy groups,

wherein at least two moles of the compound of formula I are conjugated to each mole of said polymer or macromolecular structure.

2. A method according to claim 1 wherein $R_3$ and $R_4$ are selected from alkyl, acyloxy, alkoxy, halogen, haloalkyl or cycloalkoxy.

3. A method according to claim 2 wherein $R_1$ is hydrogen.

4. A method according to claim 1 wherein said polymer or macromolecule contains the deprotonated residue of primary amino groups.

5. A method according to claim 1 wherein at least 40 moles of the compound of formula I are conjugated to each mole of said polymer or macromolecule.

6. A method according to claim 5 wherein said polymer or macromolecule is selected from the group consisting of chitosan, polyamino acids, and aminoethylcellulose.

7. A method according to claim 6 wherein said polyamioacids contain epsilon amino groups.

8. A method accroding to claim 1 wherein said polymer or macromolecule is a deprotonated residue of a polyhydroxy polymer.

9. A method according to claim 8 wherein said polymer or macromolecule is a deprotonated residue of polyvinylalcohol or of polymeric carbohydrate.